# EUROPEAN PATENT APPLICATION

(11) **EP 2 106 806 A1**
(43) Date of publication of application: **07.10.2009**
(21) Application number: 08075267.8
(22) Date of filing: 31.03.2008
(51) Int. Cl.: A61K 47/48, A61P 35/00, A61P 11/00

(54) **Nanoparticles for targeted delivery of active agents to the lung**

(71) Applicant: Fraunhofer-Gesellschaft zur Förderung der Angewandten Forschung e.V., 80686 München (DE); YISSUM RESEARCH DEVELOPMENT COMPANY OF THE HEBREW UNIVERSITY OF JERUSALEM, 91390 Jerusalem (IL)
(72) Inventor: Borlak, Jürgen, 31275 Lehrte/OT Immensen (DE); Benita, Shimon, Tel Aviv zip 64585 (IL); Harush-Frankel, Oshrat, Modi'in 71723 (IL); Debotton, Nir, Tel Aviv zip 62486 (IL); Karra, Nour, Jaffa Tel Avivi zip 68061 (IL)
(74) Representative: Baumbach, Friedrich

(57) **Abstract**

The advantages of coupling NPs containing lipophilic cytotoxic drugs with MAbs followed by local delivery to the lungs include: direct delivery to lungs, prolonged residence time in the target tissue; continuous release of significant potent drug doses at tumor sites and better cytotoxic drug internalization in tumors allowing improved efficacy. The present invention concerns a delivery system administered to the lung by inhalation comprising a polymer-based nanoparticle; and a linker comprising a first portion non-covalently anchored to said nanoparticle, wherein at least part of said first portion comprises a hydrophobic/lipophilic segment embedded in said nanoparticle; and a second portion comprising a maleimide compound exposed at the outer surface of said nanoparticle. In accordance with one embodiment, the delivery system comprises one or more targeting agents, each covalently bound to said maleimide compound. In accordance with yet another embodiment, the delivery system comprises a drug. A specific example for a linker in accordance with the invention is octadecyl-4-(maleimideomethyl)cyclohexane-carboxylic amide (OMCCA).

## Description

### FIELD OF THE INVENTION

The present invention relates to polymer-based nanoparticles for use as local delivery vehicles for the lung by inhalation.

### LIST OF PRIOR ART

The following is a list of prior art which is considered to be pertinent for describing the state of the art in the field of the invention.
Takeshi Matsuya et al. Anal. Chem. 75:6124-6132 (2003);
Terro Soukka et al. Clinical Chemistry 47(7):1269-1278 (2001)
Terro Soukka et al. Anal. Chem. 73:2254-2260 (2001);
Arai K. et al. Drug Des. Deliv. 2(2):109-120 (1987);
Harma H. et al. Luminescence 15(6):351-355 (2000);
Olivier JC. et al. Pharm. Res. 19(8):1137-1143 (2002);
Olivier JC. NeuroRx. 2(1):108-119 (2005);
Lu ZR. et al. Nature Biotechnology 17:1101-1104 (1999);
Gref R. et al. Biomaterials 24(24):4529-4537 (2003);
Nobs L. et al. Eur. J. Pharm. Biopharm. 58(3):483-490 (2004);
Ezpeleta I. et al. Int. J. Pharm. 191(1):25-32 (1999);
Lundberg BB, et al. J Pharm Pharmacol. 51(10):1099-105 (1999);
US2005/042298;
WO 1987/07150;
WO2003/088950;
US 6,221,397;
WO2005/077422.

### BACKGROUND OF THE INVENTION

"active targeting" to specific cells by attachment of specific ligands to the surface of colloidal may result in selective binding of ligands to surface epitopes or receptors on target sites [Moghimi SM, et al. Pharmacol Rev. 53(2):283-318 (2001)]. The use of monoclonal antibodies (MAb) for the treatment of cancer was suggested as a means of targeting cancer cells while sparing normal cells. MAbs are being coupled with colloidal carriers such as liposomes (to form immunoliposomes), emulsions (to form immunoemulsions) and nanoparticles (to form immunonanoparticles). These immunoconjugates thus ensure the specific recognition of the antigen site by the antibody and the release of different cytotoxic agents by the colloidal delivery system close to the inaccessible pathological target tissues, over-expressing tumor antigen. It was recently published that antibody targeting of long-circulating lipid NPs does not increase tumor localization but does increase internalization in animal models demonstrating that despite enhanced comparable localization of pegylated lipid NPs and immunolipidNPs in the target tumor, only the immunolipidNPs promoted the rapid internalization of the drug in the target cancerous cells while the lipidNPs were removed (Kirpotin *et al.,* 2006).

Immunoliposomes have already been described [Park JW, et al. J Cont Rel. 74(1-3):95-113 (2001); Park JW et al. Clin Cancer Res. 8(4):1172-81 (2002); Nam SM, et al. Oncol Res. 11(1):9-16 (1999)]. Further, it has been shown that immunoliposomes bearing polyethyleneglycol (PEG)-coupled Fab' fragments elicited prolonged circulation time and high extravasations into targeted solid tumors *in vivo* [Maruyama K, et al. FEBS Lett. 413(1):177-80 (1997)]. However, these were found to be physicochemical instable. In addition, most of these liposomal carriers were unable to incorporate significant doses of lipophilic/hydrophobic active ingredients, limiting their potential clinical efficacy. In pulmonary application specifically, potential lipid toxicity and potential lipid pneumonia are limiting the use of such carriers.

Immunoemulsions have also been described. For example, Lundberg BB et al. describes the conjugation of an anti-B-cell lymphoma monoclonal antibody (LL2) to the surface of lipid-emulsion globules by use of a poly(ethylene glycol)-based heterobifunctional coupling agent and the use of same as drug carriers [Lundberg BB, et al. J Pharm Pharmacol. 51(10):1099-105 (1999)]. Yet, lipid emulsions as such can incorporate only highly lipophilic drugs which exhibit marked poor aqueous solubility. The difficulty in retaining within the oil droplets potent moderately lipophilic cancer chemotherapy agents upon infinite dilution, limits the therapeutic applications of these dosage forms. For example, paclitaxel was found to be released rapidly form the lipid emulsion following intravenous injection [Lundberg BB. J Pharm Pharmacol. 49(1):16-20 (1997)].

A further study making use of oil emulsions involves the formation of positive oil in water emulsions; the emulsion comprising a compound presenting free NH₂ groups, at its natural state, at the oil-water interface, and an antibody, wherein the compound is linked to the antibody by a heterobifunctional linker, linking the NH₂ groups to SH groups on the antibody hinge region [Benita S. et al. International Patent Application Publication No. WO2005/077422]. Problematic issues in emulsion delivery to the lungs is potential lipid related side effects e.g. restricting the movement of cilia cells functioning in the removal of potentially harmful materials from the airways as well as possible lipid pneumonia.

Over the past few decades, there has been considerable interest in developing biodegradable and biocompatible nanoparticles (NPs) as effective drug delivery systems. Conventional NPs undergo rapid clearance following intravenous (iv) administration by the reticuloendothelial system (RES). Hydrophilic linear polyethylene glycol (PEG) molecules ranging in MW from 2000Da to 5000Da anchored on the particle surface and oriented towards the aqueous phase confer steric stabilization prevent opsonization and uptake of the NPs by the RES. These stealth NPs exhibited prolonged plasma circulating time [Avgoustakis K, et al. Int J Pharm. 259(1-2):115-27 (2003); Li Y, et al. J Control Release. 71(2):203-11 (2001); Matsumoto J, et al. Int J Pharm. 185(1):93-101 (1999); Stolnik S, et al. Pharm Res. 11(12):1800-8 (1994)].

NPs can entrap various hydrophilic and moderately lipophilic drugs such as vaccines, peptides, proteins, oligonucleotides and anti-tumor agents [Soppimath KS, J Control Release. 70(1-2):1-20 (2001); Brigger I, et al. Adv Drug Deliv Rev. 54(5):631-51 (2002)]. The encapsulation of anti-tumor agents in NPs has been widely investigated since NPs are suitable means for improving the therapeutic index of potent drugs while greatly reducing their side effects. Among the promising anti-tumor agents incorporated in NPs, doxorubicin [Soma CE, et al. J Control Release. 68(2):283-9 (2000)], irinotecan [Onishi H, et al. Biol Pharm Bull. 26(1):116-9 (2003)] and paclitaxel NPs [Xu Z et al. Int J Pharm. 288(2):361-8 (2005); Dong Y, Feng SS. Biomaterials. 25 (14):2843-9 (2004)] are exhibiting encouraging results.

Despite great clinical potential, the approach of targeting NPs to organs via MAb (immunonanoparticles) has not been fully exploited. The ability to selectively target anticancer drug loaded NPs via specific ligands against antigens over-expressed in malignant cells could improve the therapeutic efficacy of the immunonanoparticles (immunoNPs) preparations as well as reduce adverse side effects associated with chemotherapy.

Several antibodies are currently being tested for the treatment of various stages of lung cancer including different immunotoxins such as HuC242-DM4 and CMD-193 as well as monoclonal antibodies such as MORAb-003, cetuximab, panitumumab and bevacizumab which is by far the most widely used MAb in clinical trials worldwide participating in approximately 48 clinical trials in lung cancer alone (http://www.cancer.gov/lung 2007). Recently, a pivotal registration trial was conducted in China using Indium-111 labeled tumor necrosis treatment with chimeric antibodies that were given systematically or intratumorally to patients with advanced NSCLC following a failure of prior radiotherapy, or chemotherapy. The encouraging results showed 3.7% and 30.8% complete and partial patients response, respectively (Chen *et al.,* 2005)

The use of MAb as a targeting agent of drug delivery systems administered systemically has emerged during the last few years. Cirstoiu-Hapca et al. and Nobs et al have manufactured anti-HER2 and anti-CD20 mAb conjugated PLA NPs (Nobs *et al.,* 2004; Nobs *et al.,* 2006a; Nobs *et al.,* 2006b; Cirstoiu-Hapca *et al.,* 2007). The specific interaction between SKOV-3 human ovarian cancer cells (overexpressing HER2) and Daudi lymphoma cells (overexpressing CD20) and mAb-NPs was determined. The results showed selective targeting of mAb-NPs to tumor cells over-expressing the specific antigen however no toxic profiling of these NPs was performed. Olivier et al. have also synthesized immunoNPs by conjugation of an anti-transferrin receptor MAb to maleimide-grafted PEG-PLA NPs although no toxicity assessment was performed (Olivier *et al.,* 2002).

Monoclonal antibody (MAb) conjugated particulate drug delivery within the lungs may enable targeting and increased uptake to specific tissues within the lungs. As an example, omalizumab is currently used as IgE-targeted therapy approved by the United States Food and Drug Administration for asthma treatment (Kuhn, 2007).

Inhalation of nanometric biodegradable polymeric carriers can be beneficial if high local drug concentration, first pass-effect and multidrug resistance bypass can be attained. Particle deposition in the lung can be achieved with NPs although this particle size range has hardly been exploited due to possible safety issues [P.G.Rogueda, et al. Expert Opin. Drug Deliv. 4;595 (2007)]. The human lung is very attractive for systemic and local drug administration in view of the enormous adsorptive surface area (140m²) and thin adsorption mucosal membrane (0.1-0.2 µm) in the distal lung (Courrier et al. 2002).

Lung cancer is indeed one of the most common and fatal cancers worldwide. Although the present protocols of lung cancer targeting are mainly intravenously based protocols, local pulmonary drug delivery may be very attractive. However, the pulmonary delivery of NPs raises concern over increased toxicity, but also opens up the possibility for enhanced therapeutic effects and reduced dosage. Toxicity data available so far concerns mainly non-therapeutic molecules, and it remains a moot point as to whether these apply to drug molecules (Rogueda and Traini, 2007). Dailey et al studied the proinflammatory potential of biodegradable diethylaminopropylamine poly(vinyl alcohol)-graftedpoly(lactic-co-glycolic acid) (DEAPA-PVAL-g-PLGA) NPs after intratracheal instillation (Dailey *et al.,* 2006). They compared biodegradable DEAPA-PVAL-g-PLGA) NPs and non-biodegradable polystyrene NPs of various size and found out that the small biodegradable NPs presented significantly lower inflammatory response, especially in pulmonary recruitment of polymorphonuclear cells. Their conclusion was that more comprehensive toxicological studies should be designed to fully probe the safety of NPs in the respiratory tract. Though, in Dailey *et al* study as in most animal studies instillation, but not inhalation was used as a mode of delivery of NPs to lungs, the relevance of pathological observations made in animals for humans remains to be established (Medina *et al.,* 2007). Tseng et al. have performed in vivo delivery of gelatin NPs with biotinylated EGF conjugation for lung cancer targeting by aerosol administration to cancerous lung of SCID mice model, and specific accumulation in cancerous lung was confirmed by image quantification. However, in vivo toxicity issues were not addressed and NPs targeting was approved following single pulmonary delivery (Tseng *et al.,* 2007).

For example, out of a wide range of different sizes of NPs, it was shown that the 140-240 nm particles could barely be identified in the thoracic lymphatic system (Liu *et al.,* 2006). This issue is of special importance since the NPs manufactured in the present study were in size range of similar sizes. Furthermore, PLA microspheres were observed to cluster in discrete groups in the lung tissue and were not evenly distributed (Courrier *et al.,* 2002). However, this phenomenon was observed at the micrometer and not nanometeric size range scale. Major drawback of local NP therapy within the lungs is size-dependent elimination. Generally 80% of particles with a mass median aerodynamic diameter (MMAD) of less than 0.5 µm were found to be eliminated during exhalation. However, holding the breath may prevent the phenomenon (Asgharian *et al.,* 2003). Moreover, this size-dependent shortcoming can even be viewed as an advantage since these NPs appear to settle effectively within the deep lungs by means of Brownian diffusion and presents good pulmonary penetration.

Nanoparticles (NPs) have shown great potential as carrier systems for an increasing number of active molecules including hydrophobic potent cytotoxic drugs. Although capable of enhanced accumulation in the target tissue compared to plain particles, the NPs cannot provide targeting unless specific ligands such as monoclonal antibodies (MAbs) are attached to them. Coupling of MAbs to NPs is attained by covalent binding of the antibody molecule to the particle surface. These immunonanoparticles (immunoNPS) should ensure the specific recognition of the antigen site by the antibody and the release of paclitaxel palmitate, a lipophilic pro-drug of paclitaxel by the colloidal carrier close to the inaccessible pathological lung target tissues over-expressing such tumor antigens.

Aerosol therapy using particulate drug carrier systems is becoming a popular method to deliver therapeutic compounds either locally or systemically [12]. Pulmonary delivery using metered dose inhaler systems for aerosols or powders may contain nanostructures such as NPs. Research into lung delivery is driven by the potential for successful protein and peptide drug delivery by this route. Pulmonary drug delivery offers local targeting for the treatment of respiratory diseases. However, the success of pulmonary delivery of protein drugs is diminished by proteases and macrophages in the lung, which reduce their overall bioavailability, and by the barrier between capillary blood and alveolar air. Targeting mechanisms can make therefore, advantage of both, drug formulation and route of administration, and can be either passive or active. An example of passive targeting is the preferential accumulation of chemotherapeutic agents in solid tumors as a result of the differences in the vascularization of the tumor tissue compared with healthy tissue.

The interest in biodegradable particles with diameters in the nanometer range (preferably <250 nm) for pulmonary delivery has grown. It should be emphasized that up to now, to the best of our knowledge immunoNPs were not administered by aerosol therapy. It will be interesting to assess the biofate of immunoNPs as compared to NPs. However, the safety and toxicology of these systems in the lung may be problematic in part because of their extensive surface area. Different-sized "inert" particles composed of polystyrene and TiO₂ induced a surface area-dependent pulmonary-inflammatory response following their introduction to the lung. In the present study it is planned to make use of PLA/PLGA, a polymer approved for injection by the FDA for the formation of NPs and immunoNPs due to their safe biocompatible and biodegradable properties. The advantages of coupling NPs containing lipophilic cytotoxic drugs with MAbs followed by local delivery to the lungs include: direct delivery to lungs, prolonged residence time in the target tissue; continuous release of significant potent drug doses at tumor sites and better cytotoxic drug internalization in tumors allowing improved efficacy

### It should be emphasized that no published work showed the local delivery of immunonanoparticles by inhalation directly to the lungs.

### SUMMARY OF THE INVENTION

The present invention is based on the development of a simple approach for associating targeting agent, such as antibodies, to polymer-based nanoparticles (preferably those comprising a therapeutically active agent), which does not require *a priori* chemical binding of the targeting agent to the particle-forming polymer. This was achieved by the use of a molecular linker having a lipophilic portion which non-covalently anchors to the particle's polymeric matrix and a second portion comprising a maleimide compound to which it is possible in a subsequent step to bind the targeting agent. This novel approach eliminates the need to tailor for each different targeting agent a different nanoparticle composition, and enables to form a "universal" nanoparticle-linker (with an active agent such as a cytotoxic agent), which can be used to prepare different targeted systems, simply by binding to the linker different targeting agents according to needs.

Thus, according to a first of its aspects, the present invention provides a delivery system for local lung delivery by inhalation comprising:
(i) a polymer-based nanoparticle;
(ii) a linker comprising a first portion non-covalently anchored to said nanoparticle, wherein at least part of said first portion comprises a hydrophobic segment embedded in said nanoparticle; and a second portion comprising a maleimide compound exposed at the outer surface of said nanoparticle;
(iii) a drug; and
(iiii) a ligand (targeting moiety).

The nanoparticle preferably comprises an active agent carried by the particle, such as a drug, a contrasting agent and combinations of same, embedded, conjugated, impregnated, or encapsulated in said particle, or adsorbed at the surface of the particle.

The above nanoparticle-linker can be used in subsequent production of the final targeted product, as the linker is suitable for covalent binding with a targeting agent.

According to one preferred embodiment, the nanoparticle comprises one or more targeting agents each covalently bound to said maleimide compound.

The invention also provides a composition comprising the delivery system of the invention. In accordance with one embodiment, the composition comprises a pharmaceutically acceptable carrier. In accordance with some other embodiments, the composition comprises an active agent carried by said nanoparticle.

The invention also provides a method for treating or preventing a disease or disorder related to the lungs, the method comprises providing a subject in need, an amount of the delivery system of the invention by inhalation, the amount being effective to treat or prevent said disease or disorder.

Yet further, the invention provides a method of diagnosis in a subject's body a target cell or target tissue, the method comprising:
(a) providing said subject with the delivery system of the invention and carrying a contrasting agent wherein the nanoparticles are associated with one or more targeting agents effective to target said delivery system to said target cell or target tissue in the lung;
(b) imaging said contrasting agent in said body.

### BRIEF DESCRIPTION OF THE FIGURES

In order to understand the invention and to see how it may be carried out in practice, a preferred embodiment will now be described, by way of non-limiting examples only, with reference to the accompanying Figures, in which:
**Figures 1A-1C** are schematic illustrations of a delivery particle according to the invention, in which a linker (OMCCA) has a first portion anchored in the particle, and a second portion (maleimide) exposed at the surface of the particle and associated to an antibody (Y) **(****Fig. 1A****);** the delivery particle may further comprise portions of the polymer modified with polyethylene glycol **(****Fig. 1B****),** and may also carry a drug embedded in the polymeric matrix **(****Fig. 1C****).**
**Figure 2** is a three dimensional bar graph showing zeta potential measurements for non-conjugated particles (blank), trastuzumab-conjugated particles (immunoNPs), trastuzumab-conjugated and drug loaded particles (immuno DCTX NPs).
**Figures 3A-3B** shows transmission electron microscopy images of antibody- conjugated nanoparticles according to the invention, using 12nm gold labeled goat anti-human IgG, at two scales, 200 nm **(****Fig. 3A****)** and 100 nm **(****Fig. 3B****).**
**Figure 4** shows animal weight change following 3 and 14 days recovery in mice instilled with NPs and immunonanoparticles over 5 consecutive days
**Figure 5A-5D** show total BAL cells following 3 and 14 days recovery in mice instilled with NPs and immunonanoparticles over 5 consecutive days **(Fig. A),** total BAL macrophages **(Fig. B),** total BAL lymphocytes **(Fig. C)** and total BAL neutrophils **(Fig. D).**
**Figure 6:** Scoring of macrophages, congestion and inflammation within both lungs of mice installed with different formulations following 3 **(Fig. A)** and 14 **(Fig. B)** days recovery.
**Figure 7** are images of lung tissues from mice instilled with different formulations and scarified on the 8 **(Fig. A)** and 19 **(Fig. B)** days of experiment

### DETAILED DESCRIPTION OF THE INVENTION

The present invention is aimed to provide improvement of drug delivery therapy which is based on a novel one-step conjugation process of one or more targeting agents to drug-loaded nanoparticles. In particular, the invention enables the preparation of a universal nanoparticle linker (optionally in combination with a drug) that can be subsequently bound to a targeting agent of choice, so that there is no need to design a special nanoparticle for each different targeting agent. The design nanoparticles in accordance with the invention allow a better recognition of targeted cells exhibiting two surface membrane low antigen densities.

The present invention thus provides delivery systems comprising a polymer based nanoparticle and a linker comprising a first portion non-covalently anchored to said nanoparticle, wherein at least part of said first portion comprises a hydrophobic segment embedded in said nanoparticle; and a second portion comprising a maleimide compound exposed at the outer surface of said nanoparticle.

Maleimides are a group of organic compounds with a 2,5-pyrroledione skeleton as depicted in general formula (I) hereinbelow.

Maleimides are used in a wide range of applications ranging from advanced composites in the aerospace industry to their use as reagents in synthesis. For example the aerospace industry requires materials with good thermal stability and a rigid backbone both of which are provided by bismaleimides. In some applications, various linkers such as polysiloxanes and phosphonates are conjugated to the bismaleimindes to strengthen polymers made therefrom, etc.

Maleimides may also be linked to polyethylene glycol chains which are often used as flexible linking molecules to attach proteins to surfaces. The double bond readily reacts with the thiol group found on cysteine to form a stable carbon-sulfur bond. Attaching the other end of the polyethylene chain to a bead or solid support allows for easy separation of protein from other molecules in solution, provided these molecules do not also possess thiol groups.

In the context of the present invention, maleimide is conjugated to a linker to be incorporated non-covalently into a polymer based nanoparticle and the combination of the maleimide-linker with the nanoparticle provides a delivery system platform for various active agents.

The term ***"delivery system"*** which may be used herein interchangeably with the term ***"delivery nanoparticles"*** denotes physiologically acceptable, polymer-based nanoparticles which when associated with a linker, the particles have a diameter of 1 micrometer or less, preferably in the range of about 50-1000 nm, more preferably in the range of about 200-300nm. While the nanoparticles preferably have a matrix structure formed from one or more polymers. Further, while the nanoparticles may be formed from substances other than a polymer, it is to be understood that the particles are essentially polymer-based or at least their outer surface is polymer-based. Thus, the term *"**nanoparticles"*** in the context of the invention excludes liposomes or emulsion forms.

The terms ***"polymer based particles", "polymer based nanoparticles"* or *"particle-forming polymer"*** as used herein denotes any biodegradable, and preferably biocompatible polymer capable of forming, under suitable conditions, nanoparticles A variety of biodegradable polymers is available in the art and such polymers are applicable in the present invention. Approved biodegradable, biocompatible and safe polymers largely used in nanoparticle preparations are described by Gilding DK et al. [Gilding DK et al. Polymer 20:1459-1464 (1979)].

Non-limiting examples of particle-forming biodegradable polymers are polyesters such as, without being limited thereto, polyhydroxybutyric acids, polyhydroxyvaleric acids; polycaprolactones; polyesteramides; polycyanoacrylates; poly(amino acids); polycarbonates; polyanhydrides; and mixtures of same.

Preferably, the polymer is selected from polylactic acid (polylactide), polylactide-polyglycolide, polyglycolide, poly(lactide-co-glycolide), polyethylene glycol-co-lactide (PEG-PLA) and mixtures of any of same.

A further component within the delivery system is the linker comprising a first portion non-covalently anchored to the nanoparticle and a second portion comprising a maleimide compound exposed at the outer surface of said nanoparticle. The first portion is configured such that at least part of same comprises a hydrophobic segment embedded in the nanoparticle's surface.

The term ***"anchor"*** as used herein denotes the penetration of at least part of the first portion of the linker through the particle's outer surface so as to obtain a stable association between the linker and the particle. The anchoring may be achieved by the incorporation of a moiety (herein termed *"the anchor moiety"*) at the first portion of the linker which has similar physical characteristics as the polymer. Those versed in chemistry will know how to select an anchor moiety to be compatible with the substance from which the particle is essentially made. For example, when using a hydrophobic polymer to form a particle matrix, a preferred selection of an anchor moiety is a hydrophilic and/or lipophilic moiety. In other words the anchor moiety should preferably be compatible with the polymer and eventually with the incorporated drug.

The association between the anchor moiety and the particle is preferably by mechanical fixation (e.g. by embedment) of the anchor to the polymer matrix. The mechanical fixation is obtained upon formation of the particles, when using the polymer in combination with the linker during polymer solidification process. Once the polymer solidifies in the form of particulates, it "captures" the anchor moiety of the linker to form the resulting delivery system of the invention.

The linker in the context of the present invention is an amphipathic molecule, i.e. a molecule having a hydrophobic/lipophilic portion (providing the anchor) and a maleimide compound forming part of the hydrophilic portion. It is noted that in the following whenever the term *"lipophilic"* is used, it may be understood interchangeably with the term hydrophilic, as long as the hydrophobic/lipophilic moiety is compatible with the polymer forming the nanoparticle. Thus, a lipophilic portion may equally refer to a hydrophilic portion. In accordance with some embodiments, the hydrophobic/lipophilic portion comprises a hydrocarbon or a lipid comprising at least 8 carbon atoms in the hydrocarbon backbone. An exemplary range is C₈-C₃₀ carbon atoms. The lipophilic moiety may be a saturated or unsaturated hydrocarbon, linear, branched and/or cyclic.

It is noted that the linker may have one or more anchors which may be incorporated in the nanoparticle's surface. For example, a double anchor may be achieved by the use of linker comprising 1,2-Distearoyl-sn-Glycero-3-Phosphoethanolamine-N-[Maleimide(Polyethylene Glycol)2000], shown in Table 1 below, which contains two lipophilic moieties.

The linker has also a second portion to which a targeting agent (as disclosed below) binds. The binding of a targeting agent is preferably by covalent attachment, although non-covalent association may, at times, also be applicable. Covalent attachment is achieved by the inclusion in the hydrophilic portion of a chemically reactive group, in the instant invention, maleimide. Maleimide may form a stable thioether linkage with thiol groups of targeting agents.

According to some embodiments, the linker has the following general formula (I): 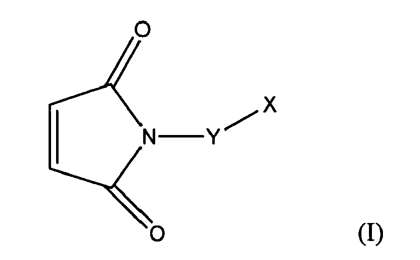 wherein

Y represents a heteroatom, a C₁-C₂₀ alkylene or alkenylene, a C₅-C₂₀ cycloalkylene or cycloalkenylene, C₆-C₂₀ alkylene-cycloalkykylene, wherein one of the carbon atoms in said alkylene or alkenylene may be replaced by a heteroatom;

X represents a carbonyl containing moiety selected from -C(O)-R₁, -C(O)-NH-R₁, -C(O)-O-C(O)-R₁, C(O)NH-R₂-R₁, or -C(O)-NH-R₂-C(O)-NH-R₁, wherein R₁ represents a hydrocarbon or a lipid comprising at least 8 carbons and R₂ represents a hydrophilic polymer.

In accordance with such embodiments, R₁ may represent a lipid; R2 a hydrophilic polymer. According to one embodiment, the lipid is selected from mono or diacylglycerol, a phospholipid, a sphingolipid, a sphingophospholipid or a fatty acid.

It is noted that R₁ should be compatible with the polymer nanoparticle matrix and should be lipophilic. In accordance with this embodiment, Y may preferably represent an alkylene-cyclohexane.

The hydrophilic polymer may be any surface modifier polymer. Polymers typically used as surface modifiers include, without being limited thereto: polyethylene glycol (PEG), polysialic acid, polylactic (also termed polylactide), polyglycolic acid (also termed polyglycolide), apolylactic-polyglycolic acid, polyvinyl alcohol, polyvinylpyrrolidone, polymethoxazoline, polyethyloxazoline, polyhydroxyethyloxazoline, polyhydroxypropyloxazoline, polyaspartamide, polyhydroxypropyl methacrylamide, polymethacrylamide, polydimethylacrylamide, polyvinylmethylether, polyhydroxyethyl acrylate, derivatized celluloses such as hydroxymethylcellulose or hydroxyethylcellulose. The polymers may be employed as homopolymers or as block or random copolymers.

Preferably, the hydrophilic polymer is polyethylene glycol (PEG). The PEG moiety preferably has a molecular weight from about 750Da to about 20,000 Da. More preferably, the molecular weight is from about 750 Da to about 12,000 Da and most preferably between about 2,000 Da to about 5,000 Da.

Preferably the polyethylene glycol is monomethoxypolyethylene glycol (monomethoxy or regular peg) Thus, a preferred lipopolymer utilized in accordance with the invention is stearylamine-monomethoxypoly(ethyleneglycol) (SA-mPEG).

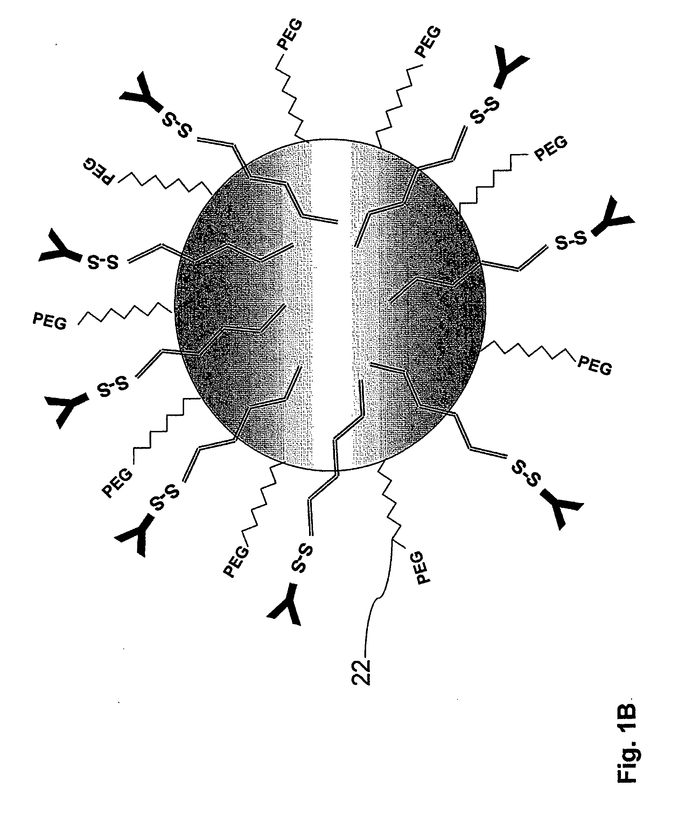

Alternatively, the hydrophilic polymer may be covalently to the polymer forming the particle, for example mPEG-polylactide, as schematically illustrated in **Fig. 1B****.**

One particular embodiment of the invention concerns a compound of formula (I) wherein Y represents an alkylene-cycloalkykylene having the formula -CH₂-C₆H₁₀-; X represents a carbonyl containing moiety having the formula -C(O)-NH-R₁, wherein R₁ is a fatty acid.

Another particular embodiment of the invention concerns a compound of formula (I) wherein the linker is selected from Octadecyl-4-(maleimidomethyl)cyclohexane-carboxylic amide (OMCCA); N-1 stearyl-maleimide (SM); succinimidyl oleate; 1,2-Distearoyl-*sn*-Glycero-3-Phosphoethanolamine-N-[Maleimide(Polyethylene Glycol)2000]; and mixtures thereof (Table 1): The chemical structures of some applicable linkers are provided in the following

**Table 1.**

**Table 1: Chemical names and structures of linkers**

| **Name** | **Structure** |
|---|---|
| Octadecyl-4-(maleimidomethyl)cyclohexane-carboxylic amide (OMCCA) | 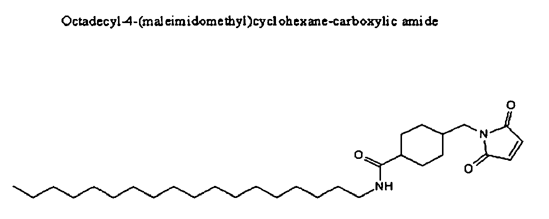 |
| Succinimidyl oleate | 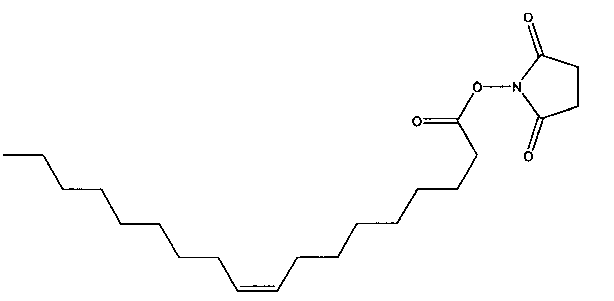 |
| Stearyl amine succinimidyl, 1,2-Distearoyl-*sn*-Glycero-3-Phosphoethanolamine-N-[Maleimide(Polyethylene Glycol)2000] | 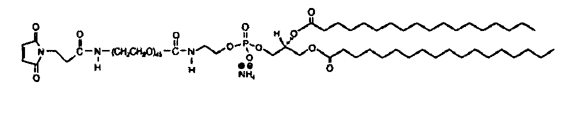 |

OMCCA, which is one preferred linker in accordance with the invention may be synthesized according to Scheme 1 below:

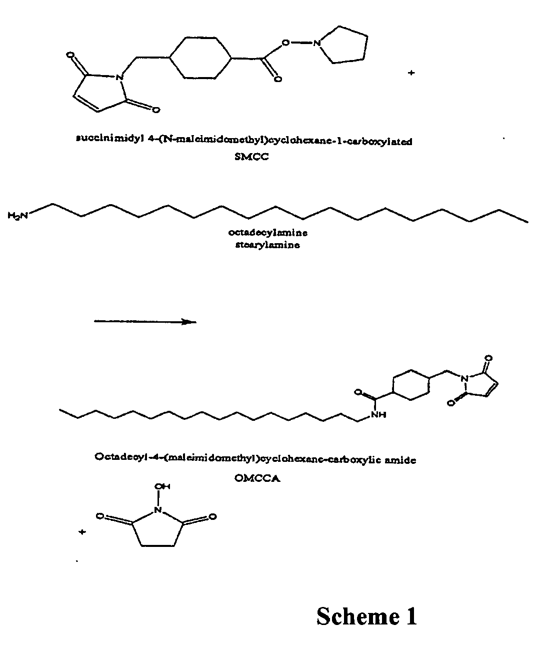

Succinimidyl oleate is commercially available from Sigma (Sigma Chemical, MO, USA; 1,2-Distearoyl-sn-glycero-3-phosphoethanolamine-N-[maleimide(polyethylene glycol)2000] is commercially available from AVANTI Polar Lipids inc, (Avanti Polar Lipids, Alabaster, AL).

The delivery system of the invention may be provided in the form of a targeted delivery system, i.e. a delivery system attached to a targeting agent. At times, when the targeting agent is an antibody or a binding fragment thereof, the targeted delivery system of the invention may be referred as *"Immunonanoparticles"*

The targeting agent may be regarded as one member of a binding couple the other member of the couple being the target on the cells, tissue to which the targeted delivery system of the invention should be selectively/ preferably delivered. The term *"binding couple"* as used herein, signifies two substances, which are capable of specifically (affinity) binding to one another. Non-limiting examples of binding couples include biotin-avidin, antigen-antibody, receptor-ligand, oligonucleotide- complementary oligonucleotide, sugar-lectin, as known to those versed in the art.

The targeting agent may be a targeting polymer or oligomer. Non-limiting examples of polymers (and immunological functional fragments thereof) comprises amino acid-based polymers (e.g. antibodies, antigens, glycoproteins), nucleic acid-based polymers (e.g. immunostimulatory oligodeoxynucleodites (ODN), sense and antisense, interference RNA (iRNA) etc. or saccharide-based polymers, such as glycoproteins (e.g. lectins).

As noted above, also fragments of any of the above targeting may be used in accordance with the invention as long as they retain their specific binding properties to the target. When the targeting agent is an antibody (see definition below), the latter may be any one of the IgG, IgM, IgD, IgA, and IgG antibody, including polyclonal antibodies or monoclonal antibodies. Fragments of the antibodies may comprise the antigen-binding domain of an antibody, e.g. antibodies without the Fc portion, single chain antibodies, fragments consisting of essentially only the variable, antigen-binding domain of the antibody, etc.

In accordance with some embodiments, the targeting agent is a low molecular weight compound such as folic acid or thiamine. For example, thiamine may be bound to the linker anchored to the polymer based nanoparticle; and the thus formed nanoparticle, will then be specifically targeted to tissues having elevated expression of the thiamine receptor. Such target cells may include cancer cells.

In some preferred embodiments, the targeting agent is a protein associated to the particle via the linker. When referring to immunonanoparticles, the targeting agent is preferably an antibody associated with the particle via covalent binding to the linker (the linker being non-covalently attached to the particle). The other member of the binding couple is an antigen to which the antibody specifically binds. As indicated above, the targeting agent may also be an immunological fragment of an antibody.

In the context of the present invention, the term ***"antibody"*** means a substantially intact immunoglobulin derived from natural sources, from recombinant sources or by the use of synthetic means as known in the art, all resulting in an antibody which is capable of binding an antigenic determinant. The antibodies may exist in a variety of forms, including, e.g., polyclonal antibodies, monoclonal antibodies, single chain antibodies, light chain antibodies, heavy chain antibodies, bispecific antibodies or humanized antibodies; as well as immunological fragments of any of the above [Antibodies: A Laboratory Manual, Cold Spring Harbor Laboratory Press, NY; Harlow et al. (1989), Antibodies: A Laboratory Manual, Cold Spring Harbor, New York ; Houston et al. (1988), Proc. Natl. Acad. Sci. USA 85: 5879-5883 ; Bird et al. (1988), Science 242: 423-426)].

As used herein, the term ***"immunological fragment"*** refers to a functional fragment of an antibody that is capable of binding an antigenic determinant. Suitable immunological fragments may be, for example, a complementarity-determining region (CDR) of an immunoglobulin light chain ("light chain"), a CDR of an immunoglobulin heavy chain ("heavy chain"), a variable region of a light chain, a variable region of a heavy chain, a light chain, a heavy chain, an Fd fragment, and immunological fragments comprising essentially whole variable regions of both light and heavy chains, such as Fv, single-chain Fv (scFv), Fab, Fab', F(ab)₂ and F(ab')₂.

According to a preferred embodiment of the invention, the antibody is a monoclonal antibody (MAb). The antibody may be a native protein or a genetically engineered product (i.e. recombinant antibody) or an antibody produced against a synthetic product.

Non-limiting examples of MAb which may be used in accordance with the invention are Bevacizumab, Omalizumab, Rituximab, Trastuzumab (all Genentech Inc.), monoclonal antibody 17-1A (EpCAM) (BD), anti-CD160 and AMB8LK (MAT Evry, France), Muromonab-CD3 (Johnson&Johnson), Abciximab (Centocor), Rituximab (Biogen-IDEC), Basiliximab (Novartis), Infliximab (centrocor), Cetuximab (Imclone Systems), Daclizumab (Protein Design Labs), Palivizumab (MedImmune), Alemtuzumab (Millenium/INEX), Gemtuzumab ozogamicin (Wyeth), Ibritumomab tiuxetan (Biogen-IDEC), Tositumomab-I131 (Corixa) and Adalimumab (Abbot).

More preferably the MAb is EpCAM. EpCAM is a MAb with high affinity towards the epithelial cell adhesion molecule, the latter over-expressed in malignant cells, such as in lung cancer cells. Thus, according to one embodiment of the invention, the delivery system may be used to delivery a cytotoxic agent to cells over expressing cell adhesion molecule.

According to some embodiments, the NP's carry two antibodies with different binding properties (e.g. different binding specificities). This structure of two different antibodies on a single nanoparticle created a "functional bispecific-like" antibody construct where the two antibodies are placed in vicinity to each other by the nanoparticle, in a relatively simple and inexpensive manner, without the need to chemically conjugate or genetically engineered a truly bi-specific single molecule

In this context, also diabodies may be used. Diabodies are a class of small bivalent and bispecific antibody fragments that can be expressed in bacteria (E.coli) and yeast (Pichia pastoris) in functional form and with high yields. Diabodies comprise a heavy (VH) chain variable domain connected to a light chain variable domain (VL) on the same polypeptide chain (VH-VL) connected by a peptide linker that is too short to allow pairing between the two domains on the same chain. This forces paring with the complementary domains of another chain and promotes the assembly of a dimeric molecule with two functional antigen binding sites. To construct bispecific diabodies the V-domains of antibody A and antibody B are fused to create the two chains VHA-VLB, VHB-VLA. Each chain is inactive in binding to antigen, but recreates the functional antigen binding sites of antibodies A and B on pairing with the other chain.

The nanoparticles of the present invention can be formed by various methods, for example: polymer interfacial deposition method, solvent evaporation, spray drying, coacervation, interfacial polymerization, and other methods well known to those ordinary skilled in the art.

Preferably the nanoparticles of the present invention are prepared by polymer interfacial deposition method as described by Fessi H et al. [Fessi H. et al. Int. J Pharm. 1989; 55: R1-R4, The nanoparticles of the present invention may be prepared as disclosed in US Pat Nos. 5,049,322 and 5,118,528].

According to the procedure by Fessi H. et al. the particle forming polymer is dissolved in a water-miscible organic solvent: such as acetone, tetrahydrofuran (THF), acetonitrile. To this polymer containing organic phase a linker as defined above is added. The resulting organic phase is then added to an aqueous phase containing a surfactant to form dispersion, following by mixing at 900 rpm, for 1 hour, and then evaporated under reduced pressure to form nanoparticles which are then washed with a suitable buffer, such as phosphate buffered saline (PBS). The organic phase may also comprise other surfactants as well as a combination of organic solvents so as to facilitate the dissolution of an active agent to be carried by the delivery system of the invention. Similarly, the aqueous phase may contain a combination of surfactants, all of which being as described by Fessi et al.

As indicated, the delivery particle preferably carries one or more active agents.
To this end, dry active agent is added to the organic phase prior to, or together with, the addition of the linker.

In order to enable formation of the nanoparticles the polymer and active agent (if incorporated) should preferably be soluble in the organic phase and insoluble in an aqueous phase, while the organic solvent and aqueous phase should be miscible.

It was found that by mere mixing the above three components, i.e. the particle forming polymer, the active agent and the linker, an amount the linker is exposed at the surface of the particle, which amount is sufficient to allow chemical binding of a targeting agent at the surface of the particles. Thus, to the forming particles (loaded with an active agent) a targeting agent is chemically associated by providing suitable conditions to allow its cross-reaction with the reactive group of the linker, exposed at the surface of the particle.

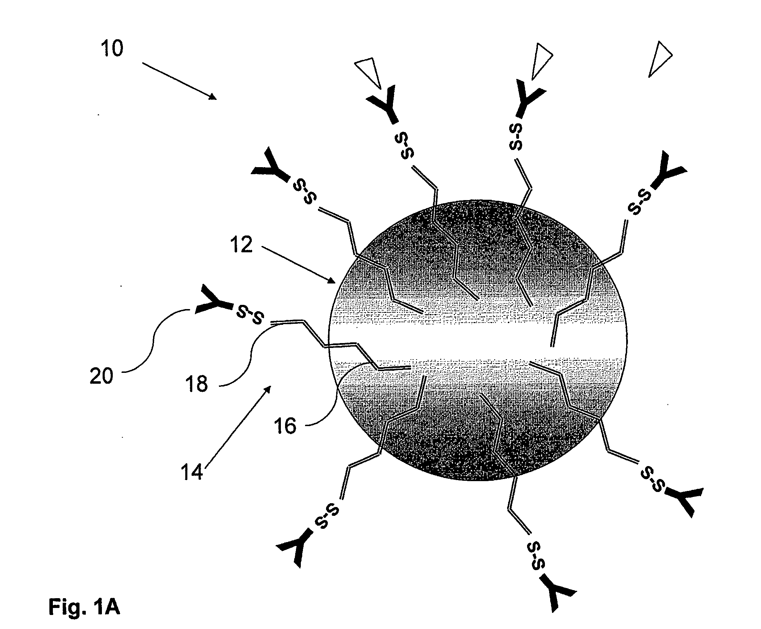

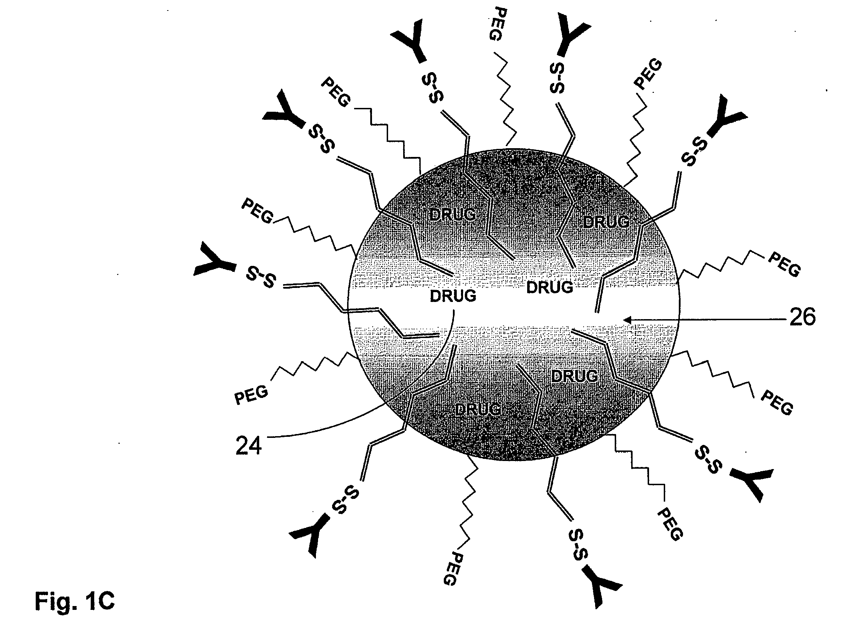

**Figs. 1A-1C** are schematic illustrations of a delivery particle according to some embodiments of the invention. Fig. **1A** provides a delivery particle **(10)** having at its outer surface **(12)** a linker **(14)** having a first portion **(16)** anchored in the particle through the outer surface, and a second portion **(18)** exposed at said surface, to which a targeting agent **(20)** is chemically bound. In this particular illustration, the linker is OMCCA, having a lipophilic anchored in the particle, and a maleimide moiety exposed at the surface. Maleimide may be chemically bound to the targeting agent via the formation of e.g. a sulfide bridge with a free thiol group at the targeting agent. **Fig. 1B** illustrates a delivery particle identical to that of Fig. **1A****,** however, having at its surface hydrophilic groups **(22),** such as PEG, to, *inter alia,* increase the circulation time of the particle in the body as appreciated by those versed in the art of drug delivery vehicles. **Fig. 1C** illustrates a delivery particle identical to that of **Fig. 1B****,** however also indicating that a drug **(24)** is embedded within the internal matrix **(26)** of the particle.

It will be appreciated that while **Figs. 1A-1C** illustrate that the first portion of the linker is fully embedded in the particle, this portion may also be partially entrapped in the particles' matrix or entrapped or encapsulated in the core. The only prerequisite is that the anchoring is essentially stable, i.e. that the linker cannot desorb from the particle.

There is a wide variety of active agents which may be carried by the delivery particle of the invention. Carrying may be achieved by conjugation or embedment of the active agent (cluster or non-clusters of the active agent) in the polymer matrix, adsorption at the surface of the particle, dispersion of the active agent in the internal space of the particle, dissolution of the active agent within the polymer forming the particle, encapsulation in the oily core of the nanoparticle etc., as known to those versed in the art.

The active agent may be a drug (therapeutic or prophylactic agent), or a diagnostic (contrasting) agent. Active agents to be administered in an aerosol formulation are preferably selected from the group consisting of proteins, peptide, bronchodilators, corticosteroids, elastase inhibitors, analgesics, anti-fungals, cystic- fibrosis therapies, asthma therapies, emphysema therapies, respiratory distress syndrome therapies, chronic bronchitis therapies, chronic obstructive pulmonary disease therapies, organ- transplant rejection therapies, therapies for tuberculosis and other infections of the lung, fungal infection therapies, respiratory illness therapies associated with acquired immune deficiency syndrome, an oncology drug, an anti-emetic, an analgesic, and a cardiovascular agent.

Anti-cancer active agents are preferably selected from alkylating agents, antimetabolites, natural products, hormones and antagonists, and miscellaneous agents, such as radiosensitizers. Examples of alkylating agents include: (1) alkylating agents having the bis-(2 chloroethyl)-amine group such as, for example, chlormethine, chlorambucile, melphalan, uramustine, mannomustine, extramustinephoshate, mechlore-thaminoxide, cyclophosphamide, if osfamide, and trifosfamide; (2) alkylating agents having a substituted aziridine group such as, for example, tretamine, thiotepa, triaziquone, and mitomycine; (3) alkylating agents of the alkyl sulfonate type, such as, for example, busulfan, piposulfan, and piposulfam; (4) alkylating N-alkyl- N-nitrosourea derivatives, such as, for example, carmustine, lomustine, semustine, or; streptozotocine; and (5) alkylating agents of the mitobronitole, dacarbazine and procarbazine type.

Examples of anti-metabolites include: (1) folic acid analogs, such as, for example, methotrexate; (2) pyrimidine analogs such as, for example, fluorouracil, floxuridine, tegafur, cytarabine, idoxuridine, and flucytosine; and (3) purine derivatives such as, for example, mercaptopurine, thioguanine, azathioprine, tiamiprine, vidarabine, pentostatin, and puromycine.

Examples of natural products include: (1) vinca alkaloids, such as, for example, vinblastine and vincristine; (2) epipodophylotoxins, such as, for example, etoposide and teniposide; (3) antibiotics, such as, for example, adriamycine, daunomycine, doctinomycin, daunorubicin, doxorubicin, mithramycin, bleomycin, and mitomycin; (4) enzymes, such as, for example, L-asparaginase; (5) biological response modifers, such as, for example, alpha-interferon; (6) camptothecin; (7) taxol; and (8) retinoids, such as retinoic acid.

Examples of hormones and antagonists include: (1) adrenocorticosteroids, such as, for example, prednisone; (2) progestins, such as, for example, hydroxyprogesterone caproate, medroxyprogesterone acetate, and megestrol acetate; (3) estrogens, such as, for example, diethylstilbestrol and ethinyl estradiol; (4) anti-estrogens, such as, for example, tamoxifen; (5) androgens, such as, for example, testosterone propionate and fluoxymesterone; (6) anti-androgens, such as, for example, flutamide; and (7) gonadotropin-releasing hormone analogs, such as, for example, leuprolide. i Examples of miscellaneous agents include: (1) radiosensitizers, such as, for example, 1,2,4-benzotriazin-3-amine 1,4- dioxide (SR 4889) and 1,2,4-benzotriazine 7-amine 1,4-dioxide (WIN 59075); (2) platinum coordination complexes such as cisplatin and carboplatin; (3) anthracenediones, such as, for example, mitoxantrone; (4) substituted ureas, such as, for example, hydroxyurea; and (5) adrenocortical suppressants, such as, for example, mitotane and aminoglutethimide.

In addition, the anticancer agent can be an immunosuppressive drug, such as, for example, cyclosporine, azathioprine, sulfasalazine, methoxsalen, and thalidomide.

Analgesic active agents, include, for example, an NSAID or a COX-2 inhibitor. Exemplary NSAIDS that can be formulated in particle of the invention include, but are not limited to, suitable nonacidic and acidic compounds. Suitable nonacidic compounds include, for example, nabumetone, tiaramide, proquazone, bufoxamac, flumizole, epirazole, tinoridine, timegadine, and dapsone. Suitable acidic compounds include, for example, carboxylic acids and enolic acids. Suitable carboxylic acid NSAIDs include, for example: (1) salicylic acids and esters thereof, such as aspirin, diflunisal, benorylate, and fosfosal; (2) acetic acids, such as phenylacetic acids, including diclofenac, alclofenac, and fenclofenac; (3) carbo- and heterocyclic acetic acids such as etodolac, indomethacin, sulindac, tolmetin, fentiazac, and tilomisole; (4) propionic acids, such as carprofen, fenbulen, flurbiprofen, ketoprofen, oxaprozin, suprofen, tiaprofenic acid, ibuprofen, naproxen, fenoprofen, indoprofen, and pirprofen; and (5) fenamic acids, such as flutenamic, mefenamic, meclofenamic, and niflumic. Suitable enolic acid NSAIDs include, for example: (1) pyrazolones such as oxyphenbutazone, phenylbutazone, apazone, and feprazone; and (2) oxicams such as piroxicam, sudoxicam, isoxicam, and tenoxicam.

Exemplary COX-2 inhibitors include, but are not limited to, celecoxib (SC-58635, CELEBREX, Pharmacia/Searle & Co.), rofecoxib (MK 966, L-74873 1, VIOXX, Merck & Co.), meloxicam (MOBIC@, co-marketed by Abbott Laboratories, Chicago, IL, and Boehringer Ingelheim Pharmaceuticals), valdecoxib (BEXTRA@, G.D. Searle & Co.), parecoxib (G.D. Searle & Co.), etoricoxib (MK-663; Merck), SC-236 (chemical name of 4-[5-(4- chlorophenyl)-3- ; (trifluoromethyl)-1H- pyrazol-1-yl)] benzenesulfonamide; G.D. Searle & Co., Skokie, IL); NS- 398 (N-(2-cyclohexyloxy-4-nitrophenyl)methane sulfonamide; Taisho Pharmaceutical Co. , Ltd., Japan); SC-58125 (methyl sulfone spiro(2.4)hept- 5-ene I; i Pharmacia/Searle & Co.); SC-57666 (Pharmacia/Searle & Co.); SC- 558 (Pharmacia/Searle & Co.); SC-560 (Pharmacia/Searle & Co.); etodolac (Lodine, Wyeth-Ayerst Laboratories, Inc.); DFU (5,5- dimethyl-3- (3-fluorophenyl)-4-(4- i methylsulfonyl)phenyl 2(5H)-furanone); monteleukast (MK-476), L-745337 ((5 methanesulphonamide-6-(2,4- difluorothiophenyl)- 1 -indanone), L-761066, L-761000, L-748780 (all Merck & Co.); DUP-697 (5-Bromo-2-(4-fluorophenyl)-3-(4 (methylsulfonyl)phenyl; DuPont Merck Pharmaceutical Co.); PGV 20229 (1-(7- tertbutyl-2,3-dihydro-3,3-dimethylbenzo(b)furan-5-yl)-4-cyclopropylbutan-1- one; Procter; & Gamble Pharmaceuticals); iguratimod (T-614; 3-formylamino-7- ] methylsulfonylamino-6-phenoxy- 4H-1- benzopyran-4-one; Toyama Corp., Japan); BF 389 (Biofor, USA); CL 1004 (PD 136095), PD 136005, PD 142893, PD 138387, and PD 145065 (all Parke-Davis/Warner- Lambert Co.); flurbiprofen (ANSAID; Pharmacia & Upjohn); nabumetone (FELAFEN; SmithKline Beecham, plc); flosulide (CGP 28238; Novartis/Ciba Geigy); piroxicam (FELDANE; Pfizer3; diclofenac (VOLTAREN and CATAFLAM, Novartis); lumiracoxib (COX-189; Novartis); D 1367 (Celltech Chiroscience, plc); R 807 (3 benzoyldifluoromethane sulfonanilide, diflumidone); JTE- 522 (Japan Tobacco, Japan); FK-3311 (4'- Acetyl-2' (2,4-difluorophenoxy) methanesulfonanilide), FK 867, FR 140423, end FR 115068 (all Fujisawa, Japan); GR 253035 (Glaxo Wellcome); RWJ 63556 (Johnson & Johnson); RWJ 20485 (Johnson & Johnson); ZK 38997 (Schering); S 2474 ((E)-(5)- (3,5-di-tert butyl-4- hydroxybenzylidene)-2-ethyl- 1,2-isothiazolidine- 1, 1 -dioxide indomethacin; I Shionogi & Co., Ltd., Japan); zomepirac analogs, such as RS 57067 and RS 104897 (Hoffmann La Roche); RS 104894 (Hoffmann La Roche); SC 41930 (Monsanto); pranlukast (SB 205312, Ono-1078, ONON, ULTAIR@; SmithKline Beecham); SB 209670 (SmithKline Beecham); and APHS (heptinylsulfide).

A description of these classes of drugs and diagnostic agents and a listing of species within each class can be found, for instance, in Martindale, The Extra Pharmacopoeia, Twenty-ninth Edition (The Pharmaceutical Press, London, 1989), which is incorporated herein by reference in its entirety. The drugs or diagnostic agents are commercially available and/or can be prepared by techniques known in the art.

Poorly water soluble drugs which may be suitably used in the practice of the subject invention include but are not limited to beclomethasone, budesonide, ciprofloxacin, cisplatin, clarithromycin, etoposide, fluconazole, itraconazole, ketoconazole, ketoprofen, methylprednisolone, , mometasone, nabumetone, norfloxacin, paclitaxel, piroxicam, triamcinolone, docetaxel, or pharmaceutically acceptable salts of any of the above- mentioned drugs.

Diagnostic agents can also be delivered by the delivery particle of the invention. Diagnostic agents may be administered alone or combination with one or more drugs as described above. The diagnostic agent can be labeled by various techniques. The diagnostic agent may be a radiolabelled compound, fluorescently labeled compound, enzymatically labeled compound and/or include magnetic compound or other materials that can be detected using techniques such as X-ray, ultrasound, magnetic resonance imaging (MRI), computed tomography (CT), or fluoroscopy.

According to one preferred embodiment the active agent to be delivered by the delivery system of the invention is a cytotoxic drug (anti-tumor agents). Cytotoxic agents exemplified herein are docetaxel, paclitaxel and paclitaxel palmitate. Specific cytotoxic agent is docetaxel (DCTX), which is known to be one of the preferred drug of choice for treating non small cell lung cancer (NSCLC).

It is appreciated that in some cases the delivery particle may comprise more than one active agent. Further, the particle may be loaded with an active agent and a suitable adjuvant therefore, i.e. an ingredient that facilitates or modified the action of the principle active agent. For example, in immunotherapy, the adjuvant will be a substance included in a vaccine formulation to enhance or modify the immune-stimulating properties of a vaccine. According to another example, the particle may comprise a combination of a drug with a multi-drug resistant (MDR) inhibitor agent to potentiate the drug action; such combination may include Verapamil known to inhibit MDR to e.g. cyclosporine A (CsA).

Further, it may occur that the targeting agent has also a therapeutic or diagnostic benefit. Thus, according to some embodiments, the particle may include only the targeting agent as the principle active agent, or in addition to the targeting agent an active agent embedded in the particle's matrix or core. Examples where the targeting agent may serves also as the active principle is trastuzumab, which is also specifically exemplified hereinbelow.

The immononanoparticles of the present invention are advantageous since they are capable of selectively binding to specific receptors or antigens and release the active agent at the desired site. The binding of the targeting agent to specific receptors or antigens triggers the transfer of the nanoparticles across biological barriers using endogeneous receptor mediated transcytosis and endocytosis systems. This will improve the therapeutic efficacy of the immunoparticles preparation when absent of the targeting agent as well as reduce adverse side effects associated with the active agent.

Nanoparticles undergo rapid clearance following IV administration by the reticuloendothelial system (RES). In order to inhibit the uptake of the nanoparticles by the RES, the nanoparticles may be modified at their surface with a hydrophilic polymer. The attachment of the hydrophilic polymer to the polymer forming the particle may be a covalent or non-covalent attachment, however, is preferably via the formation of a covalent bond to a linker anchored in the surface of the particle. The linker may be the same or different from the linker to which the targeting agent is bound. The outermost surface coating of hydrophilic polymer chains is effective to provide a particle with a long blood circulation lifetime in vivo.

According to one embodiment, the hydrophilic polymer is bound to a lipid, thus forming a lipopolymer, where the lipid portion anchors in the particle's surface.

The delivery system of the invention may be utilized for therapy or diagnosis, i.e. for targeted delivery of an active principle to a target site (cell or tissue). Thus, the invention also provides a pharmaceutical composition comprising the delivery system of the invention. According to one embodiment, the pharmaceutical composition is for the treatment or prevention of a disease or disorder, the delivery system being combined with physiologically and a pharmaceutically acceptable carrier.

The term ***"treatment or prevention"*** as used herein denotes the administering of a an amount of the active agent within the delivery system effective to ameliorate undesired symptoms associated with a disease, to prevent the manifestation of such symptoms before they occur, to slow down the progression of the disease, slow down the deterioration of symptoms, to enhance the onset of remission period of a disease, slow down the irreversible damage caused in a progressive chronic stage of a disease, to delay the onset of said progressive stage, to lessen the severity or cure a disease, to improve survival rate or more rapid recovery, or to prevent a disease form occurring or a combination of two or more of the above.

The term ***"effective amount"*** in accordance with this embodiment is an amount of the active agent embedded in the delivery particle in a given therapeutic regimen which is sufficient to treat a disease or disorder. For example, when treating cancer, the amount of the active agent, e.g. cytotoxic drug, is an amount of drug loaded delivery particles which will result, for example, in the arrest of growth of the primary tumor, in a decrease in the rate of occurrence of metastatic tumors, or a decrease in the number of metastatic tumors appearing in the individual or in a decrease in the rate of cancer related mortality. Alternatively, when the drug loaded delivery system is administered for cancer prevention, an effective amount will be an amount of said particles which is sufficient to inhibit or reduce the occurrence of primary tumors in the treated individual. The pharmaceutically *"effective amount"* for purposes herein is thus determined by such considerations as are known in the art. The amount must be effective to achieve improvement including but not limited to improved survival rate or more rapid recovery, or improvement or elimination of symptoms and other indicators as are selected as appropriate measures by those skilled in the art. For example, the amount may depend on the type, age, sex, height and weight of the patient to be treated, the condition to be treated, progression or remission of the condition, route of administration and the type of active agent being delivered.

The effective amount is typically determined in appropriately designed clinical trials (dose range studies) and the person versed in the art will know how to properly conduct such trials in order to determine the effective amount. As generally known, an effective amount depends on a variety of factors including the mode of administration, type of polymer and other components forming the nanoparticle, the reactivity of the active agent, the type and affinity of the targeting agent to its corresponding binding member, the delivery systems' distribution profile within the body, a variety of pharmacological parameters such as half life of the active agent in the body after being released from the nanoparticle, on undesired side effects, if any, on factors such as age and gender of the treated subject, etc.

In this case, for treatment purposes the drug loaded delivery particles of the invention may be administered over an extended period of time in a single daily dose (e.g. to produce a cumulative effective amount), in several doses a day, as a single dose for several days, etc. so as to prevent the damage to the nervous system.

As indicated above, the nanoparticles according to the present invention may be administered in conjunction with one or more pharmaceutically acceptable carriers. The properties and choice of carrier will be determined in part by the particular active agent, the particular nanoparticle, as well as by the particular method used to administer the composition. Preferably the route of administration of the delivery system of the invention is inhalation.

The nanoparticles can be inhaled in a solid state or dispersed in a physiologically acceptable diluent in a pharmaceutical carrier, such as a sterile liquid or mixture of liquids, including water, saline, aqueous dextrose and related sugar solutions, glycerol with or without the addition of a pharmaceutically acceptable surfactant, and other pharmaceutical adjuvants.

A person skilled in the art would readily be able to determine the appropriate concentrations of the active agent, amounts and route of administration to deliver an efficacious dosage of the active agent over time. Furthermore, one skilled in the art may determine treatment regimens and appropriate dosage using the nanoparticles of the present invention, *inter alia,* depending upon the level of control over release of the entrapped or encapsulated active agent.

Considering the above, the invention also provides a method for treating a disease related to the lung or disorder comprising administering to a subject in need an effective amount of the drug-loaded delivery system of the invention.

The types of conditions which may be treated with the delivery system of the invention are numerous, as appreciated by those versed in the art. A non-limiting list of conditions include cancer, conditions associated with the inflammatory states (inflammation or auto-immune conditions) such as infections, allergic states (e.g. bronchial asthma, drug hypersensitivity); respiratory diseases (symptomatic sarcoidosis, loeffler's syndrome, aspiration pneumonitis, tuberculosis).

In accordance with one embodiment, the invention provides a method for the treatment of cancer, by targeting, by appropriate MAbs the delivery system loaded with an anti-cancer drug (e.g. irinotecan, docetaxel and paclitaxel palmitate) to target cells in the lung.

The present invention additionally relates to a method of imaging in a subject's body a target cell or target tissue in the lung, the method comprising:
(a) providing said subject with a delivery system of the invention carrying a contrasting agent, wherein the immunonanoparticles are associated with one or more targeting agents effective to target said delivery system to said target cell or target tissue;
(b) imaging said contrasting agent in said body.

As indicated above, the delivery system of the invention may comprise a combination of a contrasting agent (imaging agent) and a therapeutic agent. Thus, by the use of the targeting system of the invention, a dual effect may be achieved, whereby the delivery of a drug may also be imaged.

The delivery device of the invention loaded with a contrasting agent may be utilized in different imaging techniques typically employed in medical diagnostics. Such include, without being limited thereto, X-ray (computer tomography (CT) of CAT scan), ultrasound, γ-scintigraphy or MRI imaging.

The contrasting agent may be any agent known in the art of imaging. An example includes, without being limited thereto, coumarin-6, fluorescent polymer conjugates such as FITC, rhodamine, gadolinium derivates.

As appreciated, while the invention is described in this detailed description with reference to pharmaceutical and diagnostic compositions, it is to be understood that also encompassed within the present invention is the use of the delivery system for other applications and in other forms.

As used in the specification and claims, the forms ***"a", "an"*** and ***"the**"* include singular as well as plural references unless the context clearly dictates otherwise. For example, the term ***"an antibody"*** includes one or more different antibodies and the term ***"a contrasting agent"*** includes one or more contrasting agents.

Further, as used herein, the term ***"comprising"*** is intended to mean that the delivery system include the recited elements, but not excluding others. The term ***"consisting essentially of"*** is used to define the delivery system that include the recited elements but exclude other elements that may have an essential significance on the treatment or imaging procedure. ***"Consisting of"*** shall thus mean excluding more than trace elements of other elements. Embodiments defined by each of these transition terms are within the scope of this invention.

Further, all numerical values, e.g. when referring the amounts or ranges of the elements constituting the device's layers, are approximations which are varied (+) or (-) by up to 20%, at times by up to 10% of from the stated values. It is to be understood, even if not always explicitly stated that all numerical designations are preceded by the term ***"about".***

### DESCRIPTION OF SPECIFIC EXAMPLES

### EXAMPLE 1-Cross-linker (OMCCA) synthesis

For the synthesis of Octadecyl-4-(maleimidomethyl)cyclohexane-carboxylic amide (OMCCA), 100mg of Sulfosuccinimidyl-4-(*N*-maleimidomethyl)cyclohexane-1-carboxylate (SMCC Pierce, IL, USA) and 80mg of stearylamine (SA, Sigma Chemical, MO, USA) were dissolved in 8ml chloroform and in 41ul of triethylamine (Reidel-de-Haen, Sigma-Aldrich Chemie GmbH, Steinheim, Germany and the reaction was incubated at 50°C for 4 hours. The solution was washed three times with 1% HCl and the chloroform was evaporated under reduced pressure. The product was desiccated overnight and weighted. The yield was about 90% and linker formation was confirmed by H-NMR (Mercury VX 300, Varian, Inc., CA, USA), IR (Vector 22, Bruker Optics Inc, MA, USA) and LC-MS (Finnigan LC*Q*DUO, ThermoQuest, NY, USA).

### H-NMR, IR and LC-MS analysis

H-NMR (of OMCCA in CDCl₃): Peaks at: 0.008, 0.849, .0893, 1.009, 1.245, 1.450, 1.577, 2.157, 2.160, 2.167, 2.173, 2.178, 2.181, 3.349, 3.372, 6.692, 7.257 ppm

IR: Peaks at: 626.89, 695.63, 722.35, 834.46, 899.52, 910.59, 934.79, 1045.94, 1120.05, 1163.30, 1214.60, 1260.82, 1362.15, 1408.40, 1431.38, 1468.04, 1541.02, 1629.86, 1701.35, 2850.80, 2923.84, 3087.43, 3318.81, 3453.91 cm⁻¹

LC-MS: Peak at: 490.17, 491.26

The analysis of the NMR and IR spectrum confirms the formation of the linker OMCCA, while the LC-MS spectra clearly corroborates the molecular weight of the product which is 490 g/mol.

### EXAMPLE 2 - polymers syntheses

### (A) PEG-PLA Synthesis and characterization

PEG-PLA (5:20) was synthesized according to well known procedure as described by Bazile D. et al. [Bazile D, et al. J Pharm Sci, 84: 493-498 (1995)]. In brief, 2 g of methoxy polyethylene glycol mw 5000 (Sigma-Aldrich Chemie GmbH, Steinheim, Germany) were mixed with 12 g of D, L -lactide (Purasorb, Purac, Gorinchem The Netherlands) for 2 hours under dried conditions at 135°C.

The polymer was analyzed by H-NMR (Mercury VX 300, Varian, Inc., CA, USA) and by differential scanning calorimetry (STARe, Mettler Toledo, OH, USA).

Diblock polyethylene glycol (mw 5000) and polylactide (mw 20000) polymer (PEG-PLA 5:20) was synthesized as described above. Gel permeation chromatography (GPC) exhibited mw of 20000 and polydispersity index [PD.I] of 1.47. The polymer was analyzed by H-NMR and by differential scanning calorimetry (DSC).

### H-NMR and DSC analysis

¹H-NMR (of PEG-PLA (5:20)): Peaks at: -0.010, -0.008, -0.001, 1.206, 1.543, 1.560, 1.567, 1.581, 1.591, 3.641, 5.136, 5.145, 5.159, 5.169, 5.182, 5.192, 5.207, 5.215, 5.231, 7.256

DSC (PEG-PLA (5:20) 3.98mg):

Peak1 : integral -118.88mJ, onset 28.70°C, peak 43.24°C, heating rate 10°C/min

Peak2: integral -1234.12mJ, onset 237.54°C, peak 273.98°C, heating rate 10°C/min

The analysis of the NMR and DSC spectrum clearly show the formation of the diblock polymer. It can be deduced that PEG is attached covalently to PLA.

### (B) Polylactide and poly(ethylene glycol-co-lactide) synthesis

The polymers: polylactide (PLA) and poly(ethylene glycol-co-lactide) (mPEG-PLA) were synthesized using the ring opening polymerization method in the presence of stannous 1-ethylhexanoate as catalyst (4). In case of synthesis of PLA; D,L-lactide (30g) and benzyl alcohol (32mg) as co-catalyst, are dissolved in 250ml of dried toluene while in the case of synthesis of mPEG-PLA; 1.5 g of methoxy polyethylene glycol (mPEG, MW 5000) was used as co-catalyst and added to 250 ml of dried toluene containing already 30 g of D,L-lactide. The refluxing mixture was stirred over a Dean-Stark apparatus over a period of 4 h for azeotropic removal of water. Stannous 1-ethylhexanoate (245mg) was added following the removal of the remaining water. Then, the mixture was heated to 135°C for 4h. The crude polymers were dissolved in methylene chloride and precipitated twice into 4 liters of cold propyl ether/petroleum ether mixture (3:2). Prior to characterization the polymers were vacuum dried. The synthesis of the co-polymer is depicted in the following Scheme 2:

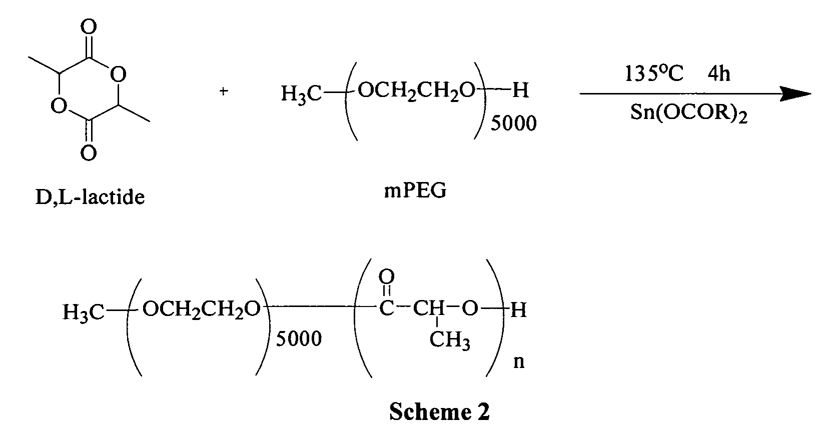

### Polylactide and poly(ethylene glycol-co-lactide) characterization

The co-polymers were characterized by gel permeation chromatography (GPC) system consisting of a Waters 1515 Isocratic high performance liquid chromatography (HPLC) pump, with 2410 refractive index detector (Waters, Milford, MA) and a Rheodyne (Cotati, CA) injection valve with a 20 µl loop. Samples were eluted with chloroform through a linear Styrogel HR column, (Waters, MA), at a flow rate of 1 mL/min. The molecular weights were determined relative to polystyrene standards (Polyscience, Warrington, PA) with a molecular weight range of 54-277.7 KDa using BREEZE 3.20 version (copyright 2000, Waters Corporation computer program). Thermal analysis was determined on a Mettler TA 4000-DSC differential scanning calorimeter (Mettler-Toledo, Schwerzzenbach, Switzerland), calibrated with Zn and In standards, at a heating rate of 20°C/min under nitrogen atmosphere. ¹H-NMR spectra (in CDCl₃) were recorded on Varian 300MHz spectrometers using TMS as internal standard (Varian Inc., Palo Alto, CA, USA).

Polymers with molecular weights in the range of 20 000-146 000 were obtained.
The basic chemical structure of PLA and mPEG-PLA polymers was confirmed by ¹H-NMR spectra which fit their composition. Overlapping doublets at 1.55 ppm are attributed to the methyl groups of the D- and L-lactic acid repeat units. The multiplets at 5.2 ppm correspond to the lactic acids CH group. When mPEG-PLA spectra is analyzed a peak at 3.65 ppm was detected which fits the methylene groups of the mPEG.

According to the data obtained from the thermographs (see Table 1), only the PEG:PLA₂₀₀₀₀ exhibited crystalline domains with the appearance of a melting point thermal event at 43.2°C. The observed crystalline domains are probably associated with the marked presence of the crystalline PEG₅₀₀₀ in the mPEG-PLA₂₀₀₀₀ co-polymer chain as suggested by the lack of melting point event in the thermographs of PLA₄₀₀₀₀, mPEG-PLA₁₀₀₀₀₀ and PLA₁₀₀₀₀₀ which show only a glass transition temperature, T_{g} (see Table 1). Indeed T_{g} increases with increase of PLA chains from 40000 to 100000 as noted in Table 1. It is well known that mPEG chains which are highly ordered elicit a crystalline character while PLA chains are less ordered exhibiting an amorphous state. This increase in PLA chains in the mPEG-PLA on the expense of PEG will increase the amorphous character of the co-polymers and consequently T_{g} will increase.

**Table 1: Physical properties of synthesized polymers**

| | **Mn^{c}** | **Mw^{c}** | **T_{g}(°C)^{b}** | **Tm(°C)^{b}** |
|---|---|---|---|---|
| **Polymer** | | | | |
| PEG:PLA₂₀₀₀₀ | 20000 | 29000 | - | 43.2 |
| PEG:PLA₄₀₀₀₀ | 37000 | 52000 | 34.1 | - |
| PEG:PLA₁₀₀₀₀₀ | 87000 | 136000 | -19.4; 49.1 | - |
| PLA₁₀₀₀₀₀ | 80000 | 83000 | 65.3 | - |

| | | | | |
|---|---|---|---|---|
| ^{a} molecular weight determined by GPC. glass transition temperature (Tg) and melting point (Tₘ) determined by DSC. ^{c}Mn is the number average of the molecular weight and Mw is the weight average of the molecular weight. | | | | |

### EXAMPLE 3

### (A) Nanoparticles (NPs) preparation and characterization

### NP's preparation

The PLA nanoparticles were prepare by the nanoparticles- polymer interfacial deposition method as described by Fessi H et al. [Fessi H, et al. Int. J. Pharm. 55: Rl-R4 (1989)]. In brief, 88 mg of the polymer PLA (polylactide, 30KDa purchased from Boehringer Ingelheim) and 38mg of the co-polymer PEG-PLA, 5:20 (polyethylene glycol of MW of 5000 and polylactide MW of 20,000) were dissolved in 20ml acetone, a water-miscible organic solvent. To this organic phase 10mg of the drug docetaxel were added. For coupling of an antibody, to the organic phase, 20mg of the linker OMCCA were added. The resulting organic phase was then added to 50ml of aqueous phase which contained 100mg *Solutol*® *HS* 15 (BASF, Ludwigshafen, Germany), as a surfactant (Macrogol 15 hydroxystearate). The dispersion was mixed at 900 rpm over 1hr and then evaporated under reduced pressure to 20ml. the NPs were washed with Phosphate Buffered Saline (PBS) 5-6 times using vivaspin 300 KDa cut-off. Spherical polymeric, nanometric (100-500nm) particles were spontaneously formed under these conditions.

**Table 2- Linker (OMCCA) containing formulation**

| **Organic phase** | **Aqueous phase** |
|---|---|
| - PEG (MW 5000)-polylactide (MW 20,000) -[PEG-PLA 5:20] 0.88% | - Solutol® HS 15^{(a)} 0.5% w/v |
| | - Water 50 ml |
| - polylactide (MW 30,000) [PLA 30] 0.19% | |
| - Acetone 20 ml | |
| - OMCCA 0.1 % w/v | |

| | |
|---|---|
| ^{(a)} Solutol^{R} HS 15 (0.5%w/v): Macrogol 15 hydroxystearate was dissolved in water at a concentration of 0.5%. | |

### Drug incorporation efficacy

Drug encapsulation (incorporation) efficacy was determined using HPLC system consisting of Kontron instruments (Watford, UK) 325 pump, Kontron instruments 332 detector adjusted at 227nm and Kontron instruments 360 autosampler. Separation was achieved by LichroCART (Merck Darmstadt, Germany) C18 (250*4 mm, 5um) column. The mobile phase was 50% acetonitrile in water at flow rate of 1 ml/min. the retention time of docetaxel was 10 minutes.

### Nanoparticle characterization

### (1) Particle size analysis

Mean diameter and particle size distribution measurements were carried out utilizing an ALV Noninvasive Back Scattering High Performance Particle Sizer (ALV-NIBS HPPS, Langen, Germany) at 25°C and using water (refractive index: 1.332; viscosity: 0.894543) as the diluent. A laser beam at 632nm wavelength was used. The sensitivity range was 0.5nm to 5µm.

### (2) Zeta potential measurements

The zeta potential of the NPs/immunoNPs was measured using the Malvern zetasizer (Malvern, UK) diluted in double distilled water.

### (3) Morphological evaluation using TEM

Morphological evaluation for the immunoNPs was performed by means of transmission electron microscopy (TEM) using gold labeled goat anti-human IgG (Jackson ImmunoResearch Laboratories, PA, USA).

Blank trastuzumab immunoNPs (containing no active ingredient) were incubated with a gold labeled anti-human IgG and negatively stained with phosphotungstic acid (PTA) 2% pH 6.4.

### Results

### Drug incorporation efficiency

The encapsulation efficiency of the cytotoxic drug docetaxel (DCTX) in the nanoparticles and in the immunonanopartricles was determined by HPLC and found to be 100% and 49%, respectively prior to purification. It was interesting to note that the theoretical drug content of the DCTX loaded NPs, 7.4%w/w (initial weight ratio PLA: PEG-PLA: DCTX; 88:38:10) was significantly higher than the drug content of DCTX immunonanoparticles, 3.3%, w/w (initial weight ratio PLA: PEG-PLA: DCTX: OMCCA; 88:38:10:20). This marked difference in DCTX content may be attributed to the presence of the linker in the polymeric matrix. During nanoparticle formation, the linker probably competes with DCTX and reduce its incorporation extent from 7.4 to 3.3%.

### Particle size analysis

The average and particle size distribution of the various NPs was measured using the ALV method. It was observed that the mean diameter of the blank NPs (containing no active ingredient) was 60nm while the diameter was 150 and 180nm for the blank immunoNPs (containing no active ingredient) and for DCTX loaded immunoNPs, respectively. The marked increase in diameter of the NPs should be related to the linker's presence which probably decreases the acetone diffusion towards the aqueous phase allowing the formation of larger NPs.

### Zeta potential measurements

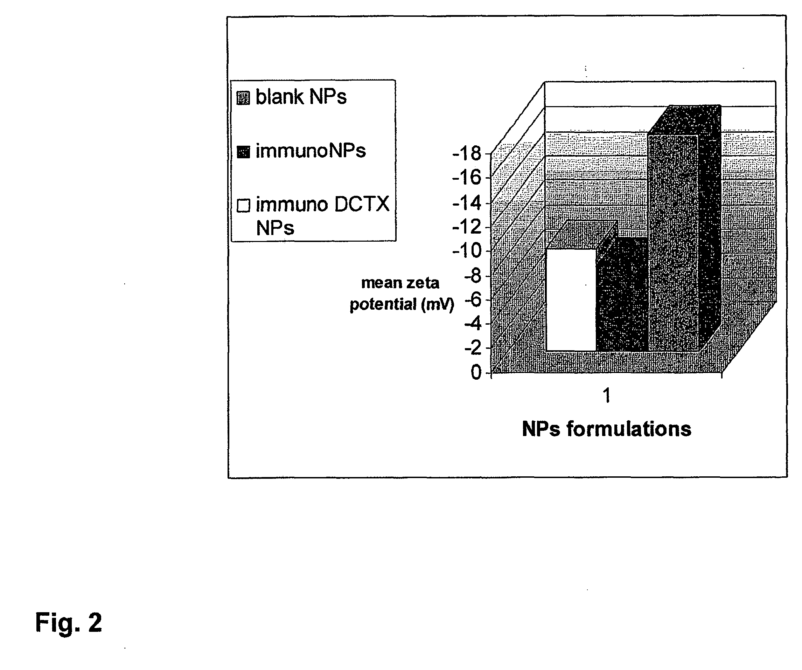

The zeta potential of the blank NPs was -18mV and decreased to -7mV for the antibody conjugates NPs (Fig. 2). The decrease in zeta potential should be attributed to the positive charge of trastuzumab at pH 7.4 since its isoelectric point is 9.

### Morphological evaluation using TEM

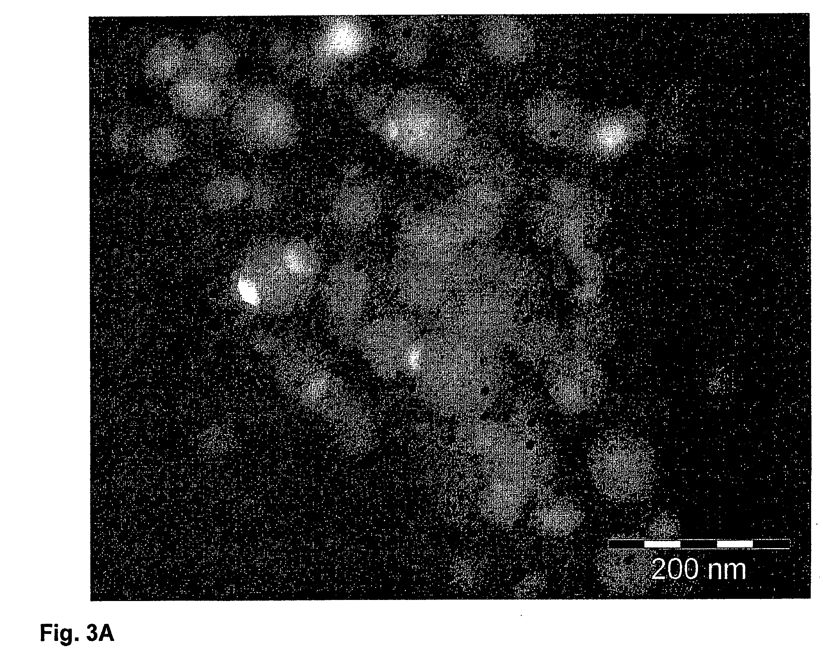

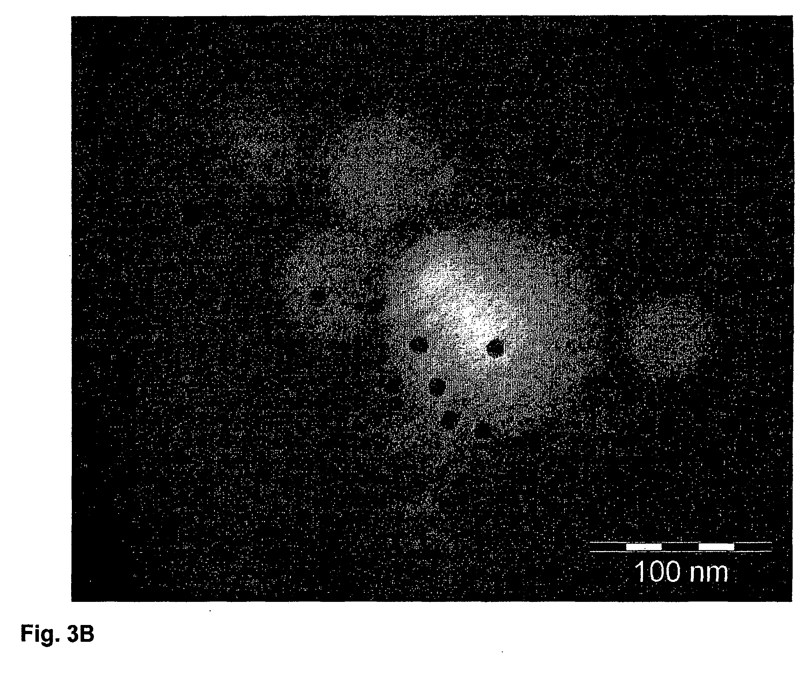

It can be noted from the results depicted in Figs. 3A-3B that each gold black spot represents one trastuzumab molecule attached to the nanoparticle surface. It can be deduced that the MAb has been efficiently conjugated to the surface of the nanoparticle by the linker and the reaction conditions did not affect the initial affinity of the MAb to the secondary antibody

### (B) Conjugation with targeting moiety

### Antibody modification for thiol groups generation

Increment of thiol groups on the MAb was preformed using the 2-iminothiolane reagent [Traut's reagent, Sigma-Aldrich Chemie GmbH, Steinheim, Germany, Traut RR, et al. Biochemistry. 12(17):3266-73 (1973); Jue R, et al. Biochemistry. 17(25):5399-406 (1978)]. Traut reagent was incubated for 45 min with purified trastuzumab at molar ratio of 30:1, respectively. The Traut modified MAb was separated on HiTrap desalting column (Amersham Bioscience, Uppsala, Sweden). Fractions containing the modified MAb were determined by UV at 280nm. Free thiol groups were determined with 5,5'-dithio-bis(2-nitrobenzoic acid) (Ellman's reagent, Sigma-Aldrich Chemie GmbH, Steinheim, Germany), by monitoring the change in absorbance at 412nm. Once reacted with Traut's reagent, mAb possess reactive sulfhydryls that can be used in conjugation protocols with sulfhydryl-reactive crosslinking reagents bearing a maleimide group such as OMCCA. The following Scheme (3) illustrates a possible conjugation reaction between reduced antibody and maleimide group of the linker:

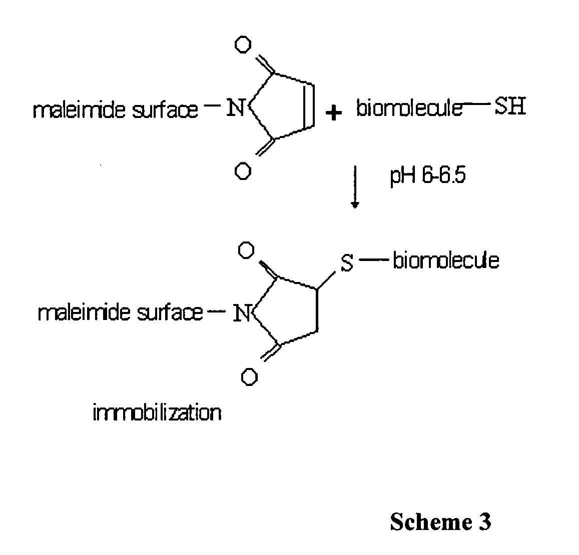

### Coupling reaction

Freshly prepared nanoparticles consisting of 88 mg of the polymer PLA (polylactide, 30KDm 38mg of the co-polymer PEG-PLA, 5:20, 0 or 10mg of the drug docetaxel and 20mg of the cross-linker OMCCA equivalent to an overall amount of blank nanoparticles of 146 mg or 156 mg of DCTX nanoparticles (PLA: PEG-PLA: DCTX: OMCCA; 88:38:0/10:20) were adjusted to pH 6.5 with 0.1N NaOH and incubated with Traut modified trastuzumab (final concentration 1mg/ml) overnight at 4°C under continuous agitation and under nitrogen atmosphere. Unreacted maleimide groups were blocked through incubation with 2-mercaptoethanol (Pierce, IL, USA) for 30min. Unconjugated antibody and 2-mercaptoethanol were separated from immunonanoparticles by gel filtration over a Sepharose CL-4B column (Amersham Bioscience, Uppsala, Sweden). Coupling efficiency was evaluated by the BCA protein assay (Bicinchoninic Acid protein assay) (Pierce, IL, USA) as described [Smith P.K., et al. Anal. Biochem. 150:76-85 (1985)].

For preparation of immunonanoparticles with various amounts of conjugated antibody, the initial ratio of Traut modified trastuzumab to maleimide-activated particles was varied. The actual investigated ratio was 146mg of blank NPs or 156mg of DCTX NPS for 26 mg of MAb.

Morphological evaluation for the final immunonanoparticles was performed by means of transmission electron microscopy (TEM) using gold labeled goat anti-human IgG (Jackson ImmunoResearch Laboratories, PA, USA).

### Drug content determination

The final drug content in the nanoparticles was evaluated as follows: the colloidal dispersion comprising a final volume of 20 ml is first ultrafiltrated using Vivaspin of 30000 daltons cutoff (Sartorius, Goettingen, Germany) to obtain 2-3 ml of clear ultrafiltrate. The concentration of DCTX in the ultrafiltrate is measured by HPLC. The remaining total volume of colloidal dispersion is then lyophilized, weighted and subjected to total DCTX content analysis using HPLC for final calculation of drug content in the nanoparticles. Various initial increasing drug ratios will be tested to identify the optimal formulation. Furthermore, the presence of possible tiny drug crystals in the colloidal dispersion will be also monitored.

### Absorption of trastuzumab to blank nanoparticles

The purpose of this determination was to evaluate whether trastuzumab molecules are physically absorbed onto blank nanoparticles, i.e. nanoparticles containing no linker anchored at their surface. To this end, 100ul (1mg) of trastuzumab 7.5mg/ml solution were mixed over 1hour at room temperature with 1 ml of blank positive and negative charged nanoparticle aqueous dispersions containing a total amount of 125mg nanoparticles. The mixture (750ul of) was then washed 5 times with 30 ml of PBS and the diluted dispersion was filtered through vivaspin 300 KDa cut-off using centrifugation (4000 rpm, 30 min) to remove unabsorbed MAb molecules.

The protein concentration was determined using PCA protein assay to detect the presence of MAb molecules in the nanoparticle supernatant.

### Results

### SH group determination

The number of sulfhydryl groups on the modified MAb was determined using Ellman's reagent compared to cysteamine as standard. The intact trastuzumab and the Traut modified trastuzumab were diluted with PBS buffer containing 0.1M EDTA pH 8 and incubated with Ellman's reagent. The Traut modified trastuzumab SH groups per MAb was determined to be 31.5 as compared to 1.4 in the intact trastuzumab.

### Coupling efficiency determination

The amount of the MAb conjugated to the NPs was determined using BCA protein assay. NPs were degraded with 0.1N NaOH at 50°C and incubated with assay reagent. The coupling efficiency for the immunoNPs (without the drug DCTX) and for the immuno DCTX loaded NPs was 71 and 77%, respectively.

### Absorption of trastuzumab to blank nanoparticles

The ratio between the amount of trastuzumab before and after separation for the positive and negative formulations was 4.2 and 2.7%, respectively.

These results ensure that there was no absorption of MAb molecules onto the nanoparticles following successive washings with PBS and therefore the coupling of MAb to linker containing nanoparticles is most probably mediated by a covalent conjugation since all the successive washings and purification processes during immunonanoparticle preparation are carried out using PBS at similar dilution extent.
The lack of MAb adsorption on vivaspin membranes was validated in previous experiments when MAb aqueous solutions were subjected to identical experimental conditions and the concentrations of MAb in the supernatant and ultrafiltrate were found to be similar.

### EXAMPLE 4

### (A) NP's preparation for in vivo study

1. Nanoparticles preparation Nanoparticles were prepared using the polymer interfacial deposition method. The organic phase contained 300 mg of the polymer mPEG-PLA MW 100,000 dalton (Sigma, St. Louis, MO), 100 mg of Tween 80 (Sigma, St. Louis, MO) and 20mg stearylamine (Sigma, St. Louis, MO) that were dissolved in 50 ml acetone (J.T. Baker, Deventer, Netherlands). The organic phase was added to 100 ml of an aqueous solution of 100 mg Solutol® HS 15 (BASF, Ludwigshafen, Germany). The suspension was stirred at 900 rpm for 1 h and subsequently concentrated by evaporation to 10 ml. All formulations were diafiltrated with a 100-ml solution of 0.1% Tween 80 (Vivaspin 300,000 MWCO, Vivascience, Stonehouse, UK) and filtered through a 1.2-µm filter (FP 30/1.2 CA, Schleicher & Schuell, Dassel, Germany). A typical cationic NP formulation consisted (in % w/w) of mPEGPLA100,000, Solutol®HS 15 1, stearylamine 0.2, Tween 80 1 and doubled-distilled water to 100. The composition of the anionic NP formulation was identical to that of the cationic NP with the exception of the cationic lipid (i.e. lacking stearylamine).
2. Immunonanoparticles preparation For preparation of immunoNPs the cross linker OMCCA (20mg) was added to the polymer within the organic phase prior to NPs formation. Rat anti mouse EpCAM antibody (IgG2Ak, batch G8.8 11/2004) was originally dissolved in Tris azide buffer in a concentration of 10mg/ml. Salts removal and dissolving in Borate buffer pH=8-8.5 was performed using nanosep centrifugal device 30K (Ann Arbor ,MI, USA). The antibody was then was mixed with 2-iminothiolane, also known as Traut's reagent (Pierce, Rockford, IL) at a molar ratio of 1:50 in Borate buffer (pH 8) for 1 h at 4°C. The solution was purified from excess Traut's reagent by gel filtration using Sephadex G-25 HiTrap desalting column (Amersham Bioscience, Uppsala, Sweden). Antibodies were collected in 0.3 ml fractions. Fractions containing mAb were determined using UV at 280 nm, pooled together and kept under nitrogen atmosphere at 4°C until coupling to anionic NPs. Freshly prepared anionic NPs were adjusted to pH 8 and incubated with Traut modified EpCAM antibody (final antibody concentration 1mg/ml) overnight at 4°C in a nitrogen environment under mild shaking. The formulation was then diafiltrated with 60 ml solution of 0.1% Tween 80 (Vivaspin 300,000 MWCO, Vivascience, Stonehouse, UK). Coupling efficiency was evaluated by the BCA protein assay. All formulations prepared were stored at 4°C in sterile glass bottles under nitrogen atmosphere. Isotonicity of the tested NP formulations was adjusted using 5% glucose.
Nanoparticles characterization by size and zeta potential determination was performed as described above.

### Results

The zeta potential and size values of different formulations are presented in Table 3. The uniformity in size of the different NPs formulations evaluated, ranging from 129 nm to 148 nm, is important since previous studies revealed surface charge dependent as well as size and surface area effects on pulmonary nanoparticulates toxicity (Tetley, 2007). The NPs size of bellow 260nm guarantees reduced uptake by alveolar macrophages (Dailey *et al.,* 2006) and utilization of the endocytic machinery for cellular internalization because of size smaller than the size of endocytic vesicle (Harush-Frenkel *et al.,* 2007). Of all the endocytic machineries that utilized in different cells types within our body, the function of pulmonary vesicular caveolae was recently discussed (Borm and Kreyling, 2004; Oh *et al.,* 2007). Vesicular caveolae within the lungs take part in transport from the luminal to the mucosal side of epithelial and endothelial cells (Borm *et al.,* 2006). Interestingly, it was shown by Harush-Frenkel et al. that caveolae may take part in the *in vitro* endocytosis of PEG-PLA NPs into epithelial MDCK cells (Harush-Frenkel *et al.,* 2007).
ImmunoNPs were manufactured by conjugation of EpCAM MAb to PEG-PLA NPs based on experience gained during the conjugation of MAbs to cationic emulsions and NPs in Prof. Benita's group. The total amount of EpCAM mAb conjugated to NPs was evaluated with the BCA protein assay kit. EpCam antibody conjugation efficiency was 77% (based on BCA in NaOH) and 80% (based on direct method BCA in DDW).

**Table 3: Physico-chemical properties of NPs and immunoNPs.**

| **Parameter** | **NPs** | **EpCAM immunoNPs** |
|---|---|---|
| **Mean diameter, nm** | **129.3** | **148.3** |
| **Mean zeta potential, mV** | **-30.7** | **-27.6** |

### (B) Safety evaluation of pulmonary delivered immunoNPs in murine model

Non invasive local pulmonary delivery was performed by means of endotracheal instillation using a mouse model. Five consecutive daily endotracheal administrations into mice lungs were performed and safety evaluation was carried out at 8 (3 days recovery) and 19 days (14 days recovery) from the first administration. During 5 days of instillation period, mice were weighed daily

The procedures involving healthy female BALB/c mice (8-10 weeks old) were approved by The Authority for Animals Facilities of The Hebrew University of Jerusalem based on guidelines from the NIH guide for the Care and Use of Laboratory Animals were followed for all experiments.

Administration was conducted via the endotracheal route using MicroSprayer™ aerosoliser (IA-1C; Penn-Century, Philadelphia, PA, USA) suitable for mice, attached to a high-pressure syringe (FMJ-250; Penn-Century) as described before (Bivas-Benita *et al.,* 2005). Prior to the procedure, mice were anaesthetized by an intraperitoneal injection of ketamine and xylazine (Fort Dodge, Iowa, USA). The method was validated following visual examination of extracted mice thorax as reported by Bivas-Benita et al.

The dose range concentration was selected based on Dailey et al. study which investigated the pro- inflammatory potential of diethylaminopropylamine polyvinyl alcohol-grafted poly(lactic-co-glycolic acid) (DEAPA-PVAL-g-PLGA) NPs instilled (and not endotracheally applied) in mice lungs

### Results

1. Method validation:
   All the mice evaluated (i.e. 10/10) for dye localization within the mice thorax immediately after endotracheal application exhibited specific lungs staining without any extra-pulmonary staining.
2. Mice survival and weight change During the 5 days of NPs application and the following 3 or 14 days recovery non of the animals died as a result from the NP application. Weight reduction or non- gaining weight is a general marker for possible toxicity. Fluctuations in weight during 5 days of instillation were observed although were not statistically significant. Non significant weight changes among the different treatments were also depicted following 3 and 14 days recovery as presented in **Fig. 4** 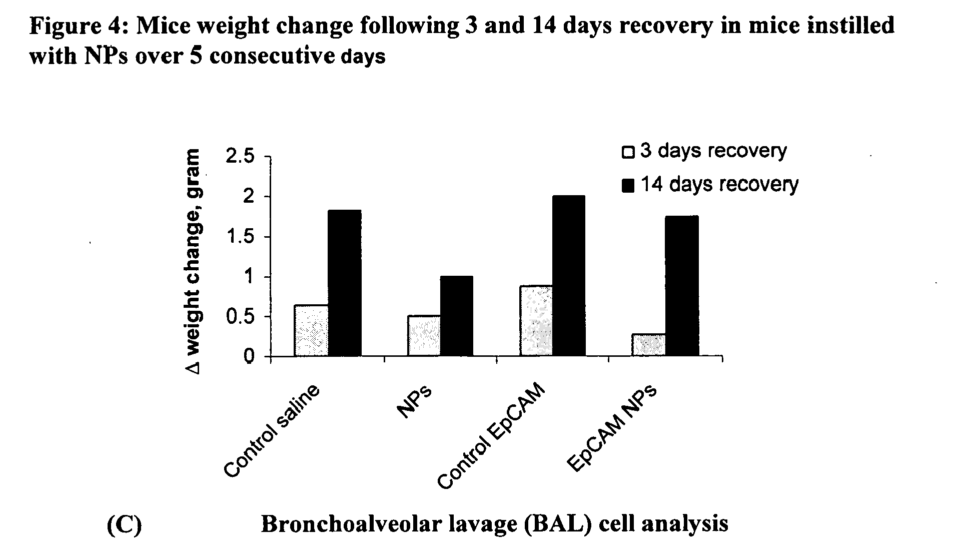
Bronchoalveolar lavage (BAL) was performed as described before (Segel *et al.,* 2005). Briefly, mice were scarified using an intraperitoneal injection of lethal dose of pentobarbital (Pental Veterinary; CTS Chemical Industries, Tel Aviv, Israel). BAL was was centrifuged for 10 min, 1000 rpm. Total BAL cell count was performed using a hemocytometer. A differential cell count was performed on 200 cells by using cytospin slides stained with Diff-Quik (Baxter). Cells numbers for the different BAL cell types are the products of the total cell count and the percentages obtained from the differential count, expressed as cells/ml of BAL fluid and analyzed for total cell count, differential cell count and macrophage

### Results

The total BAL values of PEG-PLA NPs were similar to that of the control in both 8 and 19 days experiment **(Fig 5A).** Increased macrophages levels were observed at 14 days recovery. EpCam conjugated NPs application did not result in increased total BAL cells initially and cell values were similar to that of the control and the anionic PEG-PLA NPs **(Fig 5A).** However, increased neutrophil and lymphocyte counts within the BAL were observed **(Fig 5B-D).** Furthermore, long term total cell number in BAL increased significantly compared to the saline control or the antibody saline control and also compared to the anionic PEG-PLA NPs **(Fig 5A).** Specifically, BAL macrophages, lymphocytes and neutrophils were increased (Fig **5B-D).** It appears that the effects of this formulation were noted only after 14 days recovery. It may indicate that an extended immune response took place following introduction of rat antibody to mice lungs. However, since the effects of this formulation were different from the control antibody saline, it is possible that the polymeric nature of immunoNPs conjugate may have specific effects.

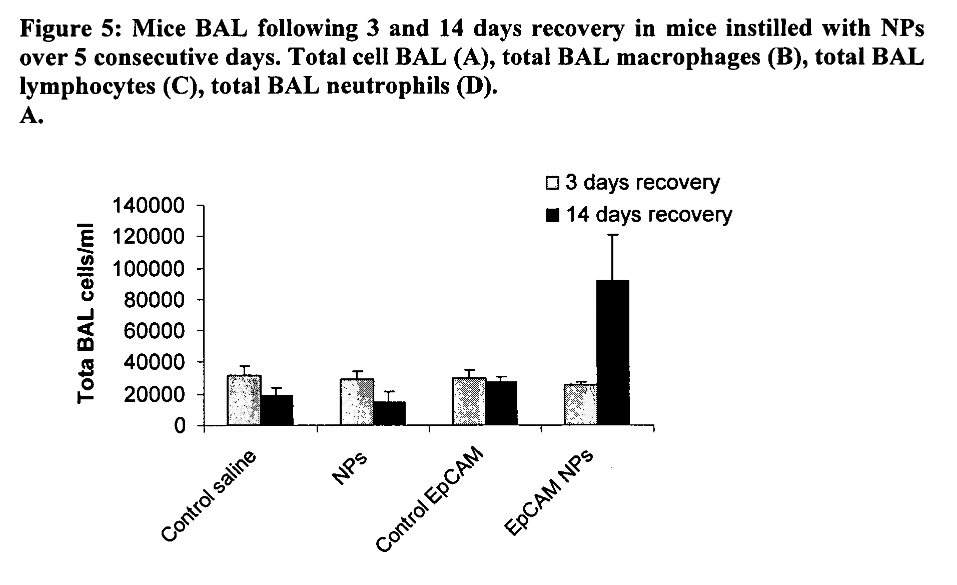

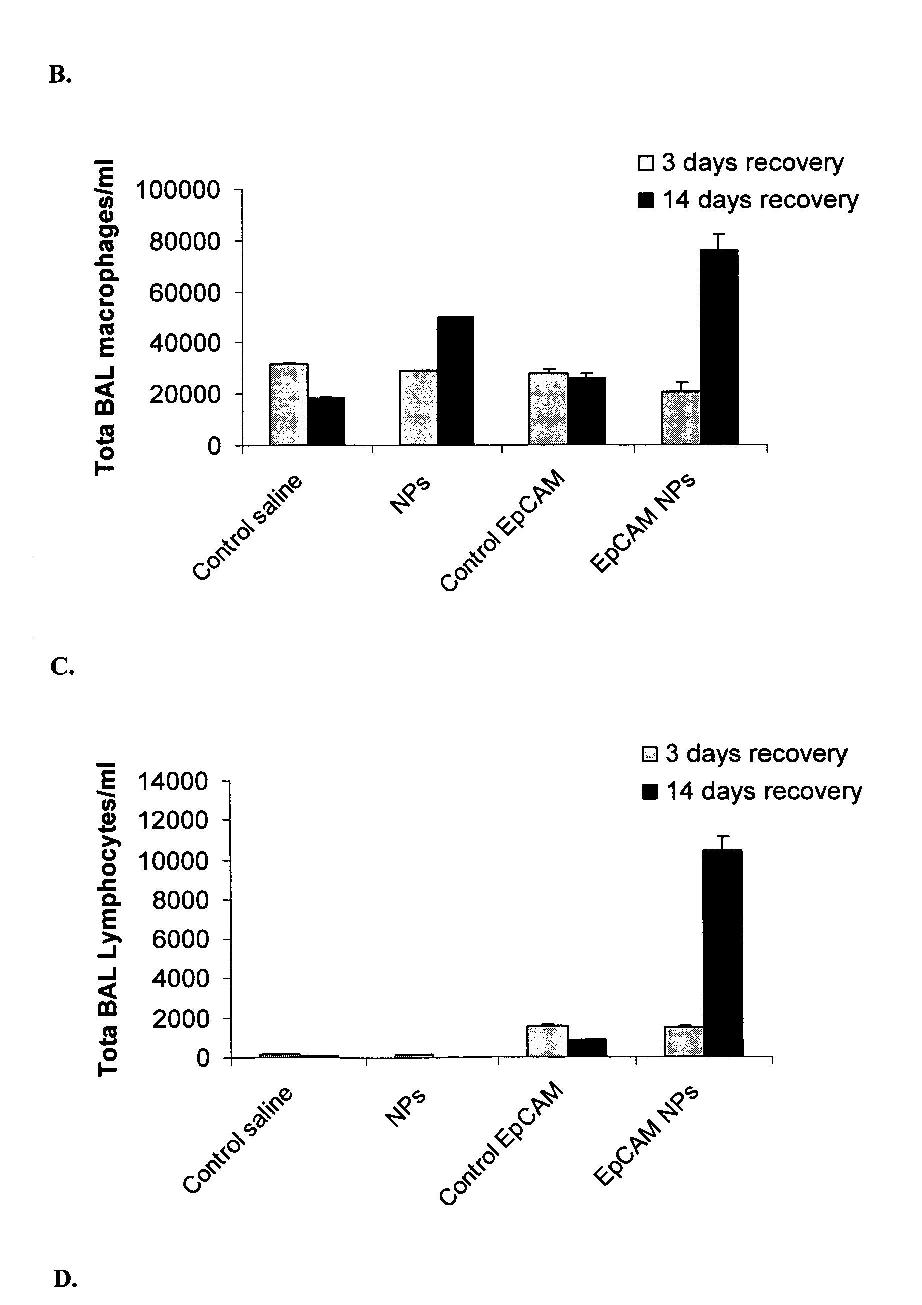

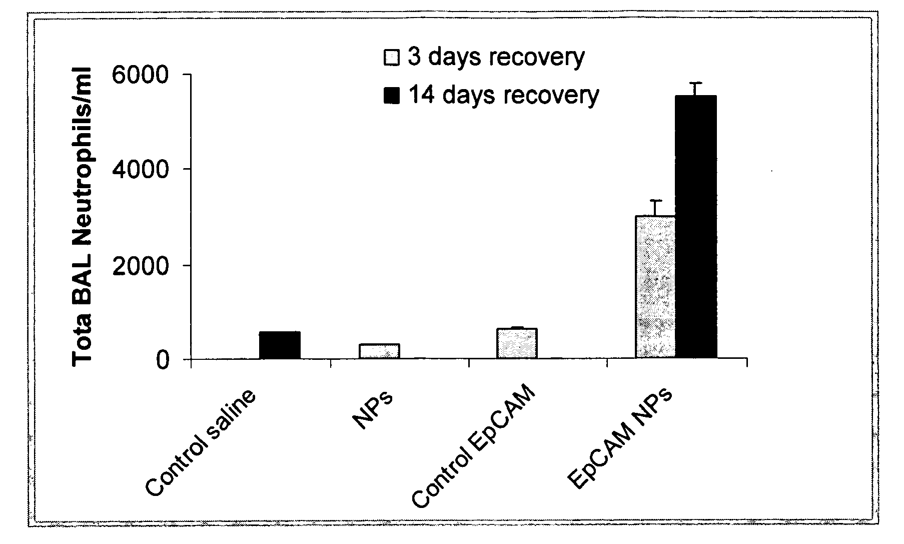

### (D) Hematological data

Following 3 and 14 days recovery blood samples were collected using a method of sub mandibular bleeding from the mice cheek pouch (Golde *et al.,* 2005). This vein was puncture and blood was collected into K3EDTA tubes (Greiner, Kremsmunster, Austria). Complete blood count was performed at the same day using sysmex K-21 blood analyzer (Sysmex Corporation, Kobe, Japan). White blood cell differential was performed manually.

### Results

The data presented in **Table 4** indicates that through 8 days of NPs application increased neutrophils and decreased lymphocyte percentage were observed, although none significantly changed compared to the other groups. The immunoNPs formulation exhibited increased stability, eosinophils and monocytes percentage as well as platelets, all within the reference value range.
Decreased platelets levels were observed during 19 days experiment for NPs **(Table 5).** NPs also exhibited the most elevated levels of eosinophils although levels were within the reference range. ImmunoNPs exhibited decrease in WBC levels compared to the controls, although non significant changes were observed among the various formulations evaluated. The different WBC populations exhibited specific increase in both neutrophils and monocytes accompanied with decreased lymphocytes for the immunoNPs group although variations were not statistically significant.

**Table 4: Total Hematological parameters following 3 days recovery in mice instilled with NPs over 5 consecutive days.**

| **Parameter Units** | **Control Saline** | **Neg NPs** | **Control EpCAM** | **EpCAM NPs** |
|---|---|---|---|---|
| | **n=6** | **n=4** | **n=7** | **n=6** |
| **WBC** | 4.45 ± 0.494 | 4.5 ± 0.993 | 4.343 ± 0.500 | 4.35 ± 0.640 |
| **10³ cmm** | 3.2-6.1 | 2.1-6.9 | 3-6.5 | 3.3-7.5 |
| **RBC** | 10.34 ± 0.18 | 9.385 ± 0.428 | 10.289 ± 0.14 | 9.77 ± 0.18 |
| **10⁶ cmm** | 9.94-11 | 8.14-9.67 | 9.92-11 | 9.13-10.3 |
| **HGB**** | 16.63 ± 0.29 | 14.58 ± 0.54 | 16.66 ± 0.09 | 15.7 ± 0.28 |
| **g/dl** | 16.2-17.7 | 13.1-15.6 | 16.5-17.2 | 14.9-16.8 |
| **HCT** | 51.93 ±1.02 | 47.8 ± 2.52 | 51.73 ± 0.73 | 48.61 ± 1.04 |
| **%** | 48.5-55 | 40.7-52.1 | 49.4-55.1 | 45.4-51.6 |
| **MCV**** | 50.05 ± 0.34 | 50.85 ± 0.57 | 50.06 ± 0.08 | 49.67 ± 0.15 |
| **fL** | 49.2-51.6 | 49.8-52.2 | 49.8-50.3 | 49.2-50.2 |
| **MCH** | 16.03 ± 0.14 | 15.55 ± 0.43 | 16.13 ± 0.20 | 16.05 ± 0.11 |
| **pg** | 15.7-16.5 | 14.3-16.1 | 15-16.7 | 15.6-16.4 |
| **MCHC**** | 32.05 ± 0.20 | 30.6 ± 0.97 | 32.23 ± 0.41 | 32.32 ± 0.29 |
| **%** | 31.3-32.6 | 27.8-32.2 | 30.1-33.6 | 31.1-33 |
| **PLAT*** | 698 ± 39 | 582 ± 188 | 918 ± 103 | 878 ± 50 |
| **10^3 cmm** | 547-765 | 360-1143 | 767-1523 | 707-1039 |
| **NEUT** | 17.83 ± 5.41 | 26.25 ± 2.50 | 22.29 ± 1.85 | 21.33 ± 3.27 |
| **%** | 7-40 | 19-30 | 15-28 | 9-31 |
| **STAB*** | 0.0 ± 0.0 | 0 ± 0 | 0 ± 0 | 0.17 ± 0.17 |
| **%** | 0-0 | 0-0 | 0-0 | 0-1 |
| **LYMP** | 81 ± 5.56 | 72.75 ± 2.14 | 76.71 ± 1.77 | 76.67 ± 3.51 |
| **%** | 59-91 | 70-79 | 72-83 | 67-90 |
| **MONO** | 1 ± 0.37 | 0.75 ± 0.48 | 0.57 ± 0.30 | 1.17 ± 0.31 |
| **%** | 0.2 | 0-2 | 0-2 | 0-2 |
| **EOS** | 0.17 ± 0.17 | 0.25 ± 0.25 | 0.43 ± 0.43 | 0.67 ± 0.21 |
| **%** | 0-1 | 0-1 | 0-3 | 0-1 |
| **BASO** | 0.0 ± 0.0 | 0 ± 0 | 0 ± 0 | 0 ± 0 |
| **%** | 0-0 | 0-0 | 0-0 | 0-0 |

**Table 5: Total Hematological parameters following 3 days recovery in mice instilled with NPs over 5 consecutive days.**

| **Parameter Units** | **Control Saline** | **Neg NPs** | **Control EpCAM** | **EpCAM NPs** |
|---|---|---|---|---|
| | **n=5** | **n=3** | **n=8** | **n=6** |
| **WBC** | 4.62 ± 0.29 | 4.30 ± 0.70 | 4.59 ± 0.73 | 4.05 ± 0.35 |
| **10^3 cmm** | 3.5-5 | 2.9-5.1 | 2.1-9 | 2.9-5.1 |
| **RBC**** | 10.1 ± 0.14 | 10.31 ± 0.66 | 9.66 ± 0.18 | 9.83 ± 0.16 |
| **10^6 cmm** | 9.66-10.3 | 9.12-11.4 | 8.61-10.2 | 9.31-10.3 |
| **HGB** | 10.17 | 16.6 ± 0.53 | 16.39 ± 0.33 | 15.42 ± 0.38 |
| **g/dl** | 15.6-16.2 | 15.6-17.4 | 14.8-17.8 | 14-16.5 |
| **HCT***** | 51.92 ± 0.79 | 56.6 ± 4.41 | 48.46 ± 1.02 | 49 ± 0.79 |
| **%** | 49.7-53.1 | 49.7-64.8 | 43-51.9 | 46-51.1 |
| **MCV***** | 51.26 ± 0.1 | 54.67 ± 1.01 | 50.1 ± 0.13 | 49.8 ± 0.18 |
| **fL** | 50.9-51.5 | 53-56.5 | 49.5-50.6 | 49.4-50.5 |
| **MCH*** | 15.62 ± 0.07 | 16.2 ± 1.10 | 16.98 ± 0.43 | 15.65 ± 0.27 |
| **Pg** | 15.4-15.8 | 15-18.4 | 15.9-19.6 | 15-16.3 |
| **MCHC***** | 30.48 ± 0.17 | 29.63 ± 2.12 | 33.9 ± 0.8 | 31.45 ± 0.50 |
| **%** | 29.9-30.8 | 26.9-33.8 | 31.9-39.3 | 30.3-32.7 |
| **PLAT** | 837 ± 26 | 549 ± 179 | 566 ± 83 | 722 ± 60 |
| **10^3 cmm** | 764-946 | 276-888 | 349-957 | 586-974 |
| **NEUT** | 21.4 ± 1.86 | 19 ± 4.04 | 17.25 ± 3.58 | 28.5 ± 1.88 |
| **%** | 14-24 | 11-24 | 8-34 | 23-36 |
| **STAB** | 0 ± 0 | 0 ± 0 | 0 ± 0 | 0 ± 0 |
| **%** | 0-0 | 0-0 | 0-0 | 0-0 |
| **LYMP** | 77.6 ± 1.69 | 79 ± 3.51 | 81.75 ± 3.80 | 69.83 ± 1.83 |
| **%** | 74-84 | 75-86 | 65-92 | 62-75 |
| **MONO** | 0.6 ± 0.4 | 1.33 ± 0.7 | 0.63 ± 0.32 | 1.33 ± 0.42 |
| **%** | 0-2 | 0-2 | 0-2 | 0-3 |
| **EOS** | 0.4 ± 0.24 | 0.67 ± 0.33 | 0.38 ± 0.18 | 0.33 ± 0.21 |
| **%** | 0-1 | 0-1 | 0-1 | 0-1 |
| **BASO** | 0.0 ± 0 | 0±0 | 0 ± 0 | 0 ± 0 |
| **%** | 0-0 | 0-0 | 0-0 | 0-0 |

### (E) Histopathology

Samples of both lungs, the heart, liver and kidney were preserved in formaldehyde. Each vial was assigned a number, with the treatment unknown to the pathologist. The lungs were fixed for at least 7 days before further processing. The formalin-fixed samples were embedded in paraffin, thin-sectioned, and mounted on glass microscope slides using standard histopathological techniques. Sections were stained with hematoxylin-eosin and examined by light microscopy.

### Results

No pathologic changes were found in extrapulmonary organs i.e. heart, liver and kidney, in both short and long term experiments. Triplicates of both lungs were evaluated separately with no significant changes observed between both lungs. The pathological changes in the lung samples examined were subtle and minimal. The main finding was that of reactive macrophages in the alveolar spaces. The differences between the very small numbers of macrophages if present at all, in the various groups were barely detectable so that exact objective quantification was not practical and not possible. The same appears to the attempts of quantifying and defining the subtle differences in the very moderate findings of inflammation and congestion. However, ranking of inflammation, congestion and macrophages within lungs was performed **(Fig 6 A and B)** It can be observed that following 3 days recovery few macrophages were indeed observed in 10-20% of the alveoli within the lungs of mice applied with NPs, immunoNPs and EpCAM control and these level of macrophages remained similar only for immunoNPs during the long term experiment. Interestingly, NPs exhibited also relatively high scores of inflammation and congestion in short term experiment that were reduced to normal values through the additional 14 days of recovery. Only for immunoNPs formulation increased inflammation score was observed compared to the controls during the 19 days of experiment. Representative pictures of each group are also presented **(Fig 7 A and B).**

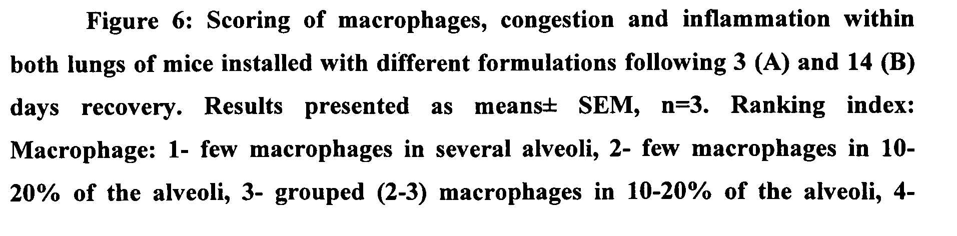

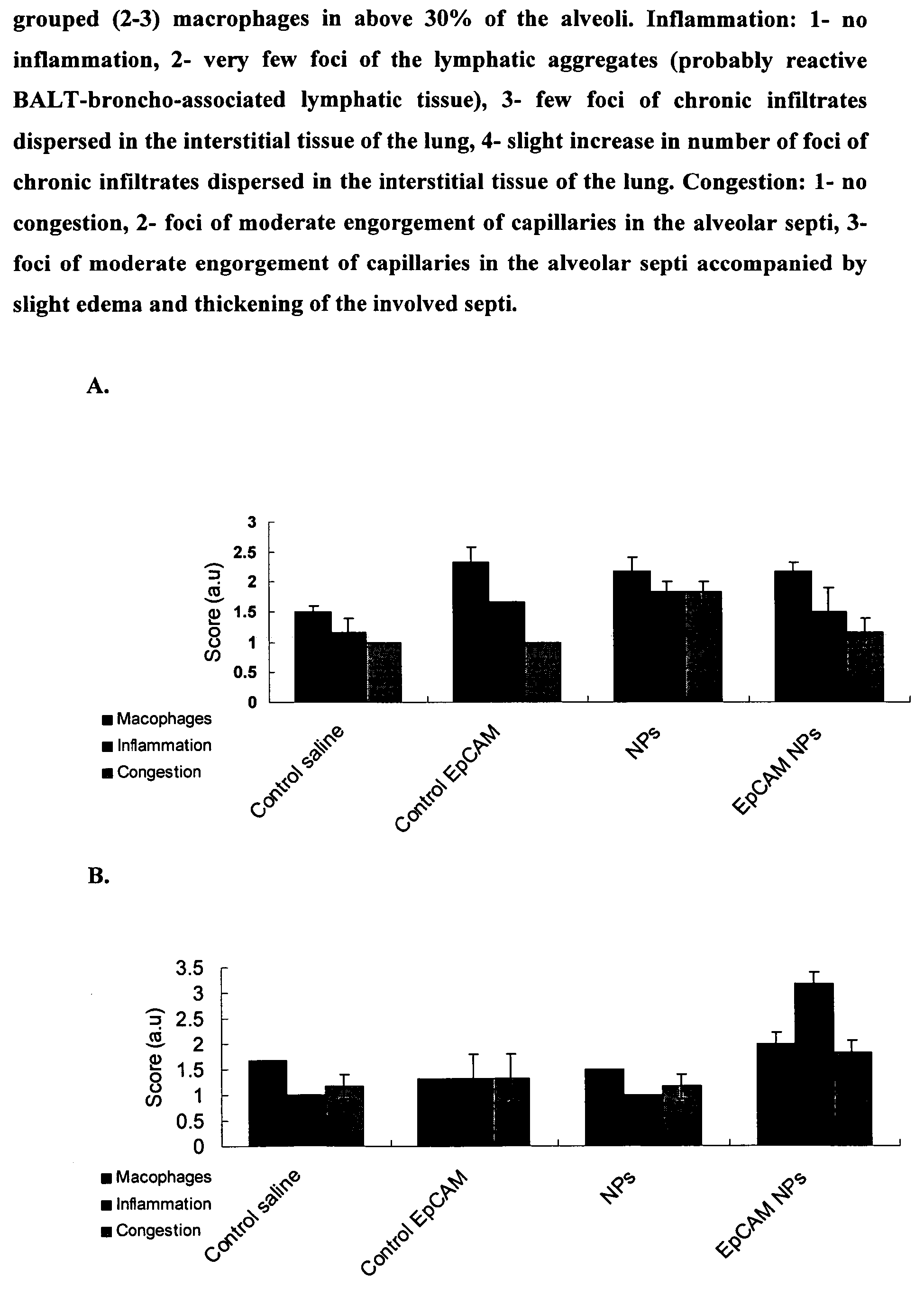

### Figure 7: Lung tissues from mice instilled with different formulations and scarified on the 8 (A) and 19 (B) days of experiment. Magnifications of pictures presented are X 400. The pictures presented: a. control saline, b. PEG-PLA NPs, c. control EpCAM saline and e. EpCAM NPs. A.

### B.

The overall results clearly indicated that immunonanoparticles can be used for local delivery to the lung by inhalation and can elicit a therapeutic activity once used in a lung cancer diseased animal model.

## Claims

1. A delivery system for local lung delivery by inhalation comprising:
(i) a polymer-based nanoparticle;
(ii) a linker comprising a first portion non-covalently anchored to said nanoparticle, wherein at least part of said first portion comprises a lipophilic segment embedded in said nanoparticle; and a second portion comprising a maleimide compound exposed at the outer surface of said nanoparticle;
(iii) a drug; and
(iiii) a ligand (targeting moiety).

2. The delivery system of Claim 1, wherein said linker is an amphipathic molecule.

3. The delivery system of Claim 1 or 2, wherein said lipophilic portion comprises a hydrocarbon or a lipid comprising at least 8 carbons.

4. The delivery system of any one of Claims 1 to 3, wherein said linker has the following general formula (I): 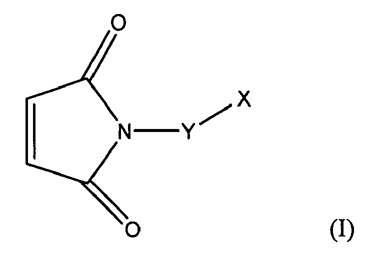 wherein
**Y** represents a heteroatom, a C₁-C₂₀ alkylene or alkenylene, a C₅-C₂₀ cycloalkylene or cycloalkenylene, C₆-C₂₀ alkylene-cycloalkykylene, wherein one of the carbon atoms in said alkylene or alkenylene may be replaced by a heteroatom;
X represents a carbonyl containing moiety selected from -C(O)-R₁, -C(O)-NH-R₁, -C(O)-O-C(O)-R₁, C(O)NH-R₂-R₁, or -C(O)-NH-R₂-C(O)-NH-R₁, wherein R₁ represents a hydrocarbon or a lipid comprising at least 8 carbons and R₂ represents a hydrophilic polymer.

5. The delivery system of Claim 4, wherein said R₁ is a lipid selected from mono or diacylglycerol, a phospholipid, a sphingolipid, a sphingophospholipid or a fatty acid.

6. The delivery system of Claim 4 or 5, wherein said Y is an alkylene-cyclohexane.

7. The delivery system of Claim 6, wherein said Y represents an alkylene-cycloalkykylene having the formula -CH₂-C₆H₁₀-; X represents a carbonyl containing moiety having the formula -C(O)-NH-R₁, wherein R₁ is a fatty acid.

8. The delivery system of any one of Claims 1 to 7, wherein said linker is selected from Octadecyl-4-(maleimidomethyl)cyclohexane-carboxylic amide (OMCCA); N-1 stearyl-maleimide (SM); succinimidyl oleate; 1,2-Distearoyl-sn-Glycero-3-Phosphoethanolamine-N-[Maleimide(Polyethylene Glycol)2000]; and mixtures thereof.

9. The delivery system of Claim 6 or 7, wherein said linker is octadecyl-4-(maleimideomethyl)cyclohexane-carboxylic amide (OMCCA).

10. The delivery system of any one of Claims 1 to 9, comprising an active agent.

11. The delivery system of Claim 10, wherein said active agent is embedded, impregnated or encapsulated in said particle, or adsorbed to the surface of the particle.

12. The delivery system of any one of Claim 1 to 11, wherein said polymer is a biodegradable polyester selected from polyhydroxybutyric acid, polyhydroxyvaleric acid, polycaprolactone, polyesteramide, polycyanoacrylate, poly(amino acids), polycarbonate, polyanhydride, poly alkylcyanoacrylate and mixtures of same.

13. The delivery system of Claim 12, wherein said polyester is polylactide (PLA), polyglycolide, polylactide-polyglycolide, poly(lactide-co-glycolide) or polyethylene glycol-co-lactide (PEG-PLA).

14. The delivery system of Claim 4, wherein said hydrophilic polymer is selected from polyethylene glycol (PEG), polysialic acid, polylactic (also termed polylactide), polyglycolic acid (also termed polyglycolide), apolylactic-polyglycolic acid, polyvinyl alcohol, polyvinylpyrrolidone, polymethoxazoline, polyethyloxazoline, polyhydroxyethyloxazoline, polyhydroxypropyloxazoline, polyaspartamide, polyhydroxypropyl methacrylamide, polymethacrylamide, polydimethylacrylamide, polyvinylmethylether, polyhydroxyethyl acrylate, derivatized celluloses such as hydroxymethylcellulose or hydroxyethylcellulose.

15. The delivery system of Claim 14, wherein said hydrophilic polymer is PEG having an average molecular weight in the range between 2,000 and 5,000Da.

16. The delivery system of any one of Claims 10 to 15, wherein said active agent is a drug, a contrasting agent or a mixture of same.

17. The delivery system of any on of Claims 1 to 16, comprising one or more targeting agents, each covalently bound to said maleimide compound.

18. The delivery system of Claim 17, wherein said targeting agent is a polymer selected from an amino acid-based, nucleic acid-based, or saccharide based polymer and combination of same.

19. The delivery system of Claim 18, wherein said targeting polymer is selected from ligands, antibodies, antigens, glycoproteins.

20. The delivery system of any one of Claims 17 to 19, wherein said targeting agent is a low molecular weight ligand.

21. The delivery system of Claim 19, wherein said antibody is a mono- or polyclonal antibody (MAb).

22. The delivery system of Claim 21, wherein said MAb is a native or genetically engineered antibody.

23. The delivery system according to claim 18 or 22, comprising at least two antibodies or antibody fragments, each with different binding specificity.

24. The delivery system of any one of Claims 21 to 23, wherein said genetically engineered antibody is trastuzumab, AMB8LK, Ep-CAM or a combination of two of them.

25. A composition comprising the delivery system of any one of Claims 1 to 24 in combination with a pharmaceutically acceptable carrier.

26. A method for treating or preventing a disease or disorder related to the lung, the method comprises providing a subject in need, an amount of the delivery system of any one of Claims 1 to 24, or of a composition according to 26, wherein said delivery system comprises a drug, the amount of the drug being effective to treat or prevent said disease or disorder.

27. A method of imaging in a subject's body a target cell or target tissue, the method comprising:
(a) providing said subject with a delivery system according to any one of Claims 1 to 24 carrying a contrasting agent, wherein the nanoparticles are associated with one or more targeting agents effective to target said delivery system to said target cell or target tissue;
(b) imaging said contrasting agent in said body.

28. The method of Claim 25, wherein said contrasting agent is coumarin-6.

29. The method of Claim 25, wherein said delivery system comprises a drug embedded in said particle.

30. The method of Claim 29, wherein said drug is a cytotoxic drug.

31. The method of claim 30, wherein said cytotoxic drug is an anti-cancer drug selected from docetaxel and paclitaxel palmitate.
